(19) Europäisches Patentamt
European Patent Office
Office européen des brevets

(11) EP 4 433 109 B1

(12) **EUROPEAN PATENT SPECIFICATION**

(45) Date of publication and mention of the grant of the patent:
**04.02.2026 Bulletin 2026/06**

(21) Application number: **22821329.4**

(22) Date of filing: **17.11.2022**

(51) International Patent Classification (IPC):
***A61L 31/04*** *(2006.01)* ***A61L 31/14*** *(2006.01)*

(52) Cooperative Patent Classification (CPC):
(C-Sets available)
**A61L 31/04; A61L 31/148;** A61L 2400/18; A61L 2430/36 (Cont.)

(86) International application number:
**PCT/EP2022/082260**

(87) International publication number:
**WO 2023/089021 (25.05.2023 Gazette 2023/21)**

(54) **BIOABSORBABLE URETHRAL STENT**

BIOABSORBIERBARER HARNRÖHRENSTENT

ENDOPROTHÈSE URÉTRALE BIOABSORBABLE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **17.11.2021 EP 21383044**

(43) Date of publication of application:
**25.09.2024 Bulletin 2024/39**

(73) Proprietor: **Polimerbio, S.L.**
**20014 Donostia - San Sebastián (ES)**

(72) Inventors:
- **FERNÁNDEZ MUÑOZ, Antonio**
  **20014 San Sebastián (ES)**
- **GEIJO ARENAL, David**
  **20014 San Sebastián (ES)**
- **FERNÁNDEZ HERNÁNDEZ, Jorge**
  **20014 San Sebastián (ES)**
- **ANTIGÜEDAD AMO, Juan Carlos**
  **20014 San Sebastián (ES)**
- **RUBIO EMAZABEL, Laura**
  **20014 San Sebastián (ES)**
- **POLO ARROYABE, Yurena**
  **20014 San Sebastián (ES)**

(74) Representative: **Aera A/S**
**Niels Hemmingsens Gade 10, 5th Floor**
**1153 Copenhagen K (DK)**

(56) References cited:
**EP-A1- 0 943 299**
**WO-A1-2010/132899**
**US-A1- 2013 331 927**
**US-A1- 2016 081 827**
**US-B2- 8 784 465**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 433 109 B1

(52) Cooperative Patent Classification (CPC): (Cont.)

C-Sets
**A61L 31/04, C08L 67/04**

## Description

### FIELD OF THE INVENTION

The present invention concerns a medical device which can be used to prevent the obstruction of a body lumen, such as the urethra. Advantageously, the stent can be inserted after a surgical procedure to reduce stricture and because the stent is bioabsorbable, a second surgery can be avoided to remove the stent once it has fulfilled its function.

### BACKGROUND OF THE INVENTION

Urethral stricture is an abnormal narrowing of the urethra that can block the flow of urine. This is produced by the generation of fibrous scar tissue along the duct, called ischemic spongiofibrosis and is usually caused by an attack on the urethra or its mucosa, derived from a traumatic rupture, infection, congenital, previous surgery (catheters, catheterizations...), swelling, after another illness, or from a growing tumor near the urethra.

Urethral stricture has a prevalence of around 0.6% of the adult male population (229-627 patients out of 100,000 males), with iatrogenesis (damage caused by a medical act of other types of operations) being the main cause of the condition (etiology), as well as traumatic. This prevalence is increasing due to the aging of the population, since the average patient is between 50 and 60 years old. Its treatment requires surgery that can be performed by:

- Urethrotomy → It is used for small (<3 cm) and longitudinal strictures, especially for young patients. It is performed using a urethrotome (endoscopy) that incorporates a knife that cuts the affected tissue. After the operation, a guide tube must be left between 1 and 4 weeks, which facilitates the urinary tract and healing. Subsequently, it is extracted.

- Urethroplasty → It is the gold-standard technique, but it is complex since it requires a cut and open surgery from the perineum to locate and remove the stenosis. It is used for large strictures and urethral regeneration is necessary by re-epithelialization of the urethra from healthy mucosa (obtained from other parts of the body, for example, the mouth) that is joined with sutures. Subsequently, a non-biodegradable guide catheter (must also be placed during the same period as in the previous case.

Foley catheter are used for this type of intervention and as guide tubes. They are flexible latex tubes that are introduced to the bladder, where a balloon is inflated with sterile water for its correct location and they serve to support the urethra and facilitate the transit of urine, while re-epithelialization occurs. Currently, the tube is usually coated manually with antifibrotic and antibacterial medications to prevent poor healing or reduce the risk of infection.

On the other hand, on the market there are non-bioabsorbable intraurethral stents such as Urolume (metallic) which, according to several analyzed articles, has generated problems of fibrosis, infection, complexity of the operation etc., which makes its use inadvisable in many cases. The same happens with others like Memokath or Allium URS. On the other hand, the American company Cook Medical has a family of urethral stents designed for hypospadias, which are expelled once the suture is absorbed during urination. Other companies, such as SRS Medical, have developed alternative stents to Foley catheters, but they are not bioabsorbable either.

EP 0 943 299 discloses stents based on bioabsorbable aliphatic polyesters and possibly combinations of such polyesters. No specific combinations are disclosed, however. Furthermore, no examples of actual bioabsorption are provided.

US 2013/331927 A1 discloses a stent suitable for insertion into a body lumen such as the urethra, comprising a copolymer of racemic L,D-lactide and ε-caprolactone. The stent expands into a rigid form and shape after insertion into a body lumen.

There is therefore still a need for tubular constructs suitable for insertion into a body lumen, such as the urethra, having the right mechanical properties (flexibility / stiffness, elasticity etc.) as well as being bioabsorbable at a time scale compatible with the healing time of e.g. urethral stenosis removed by surgery.

### SUMMARY OF THE INVENTION

In a first aspect, the present invention concerns a tubular construct suitable for insertion into a body lumen, such as the urethra, said construct comprising a polymeric material prepared by co-polymerization of ε-caprolactone, ε-decalactone, δ-valerolactone, ethylene brassylate, or δ-hexalactone with lactide. In a further aspect, the present invention concerns a polymeric material prepared by co-polymerization of ε-caprolactone, ε-decalactone, δ-valerolactone, ethylene brassylate, or δ-hexalactone with lactide for use in the prevention of obstruction of a body lumen, such as the urethra.

**BRIEF DESCRIPTION OF THE FIGURES**

FIGS. 1A-1B illustrate a perspective view (1A) and longitudinal view (1B) of a tubular construct without any protrusions,

FIGS. 2A-2C illustrate an example tubular construct according to the disclosure,

Figs. 3A-3B illustrate a perspective view (3A) and a side view (3B) of an example tubular construct according to the disclosure,

Figs. 4A-4B illustrate a perspective view (4A) and a side view (4B) of an example tubular construct according to the disclosure,

Figs. 5A-5B illustrate a perspective view (5A) and a side view (5B) of an example tubular construct according to the disclosure,

Figs. 6A-6B illustrate a perspective view (6A) and a side view (6B) of an example tubular construct according to the disclosure,

Figs. 7A-7B illustrate a perspective view (7A) and a side view (7B) of an example tubular construct according to the disclosure,

Figs. 8A-8B illustrate a perspective view (8A) and a side view (8B) of an example tubular construct according to the disclosure,

Figs. 9A-9B illustrate a perspective view (9A) and a side view (9B) of an example tubular construct according to the disclosure,

Figs. 10A-10B illustrate a perspective view (10A) and a side view (10B) of an example tubular construct according to the disclosure,

Figs. 11A-11B illustrate a perspective view (11A) and a side view (11B) of an example tubular construct according to the disclosure,

Fig. 12 illustrates a schematic example of a plurality of protrusions according to the disclosure,

Figs. 13A-13B illustrate a schematic example of a plurality of protrusions according to the disclosure,

Figs. 14A-14B illustrates a schematic example of a plurality of protrusions according to the disclosure,

Figs. 15A-15B illustrate a perspective view (15A) and a side view (15B) of an example tubular construct according to the disclosure,

Figs. 16A-16B illustrates a schematic example of a plurality of protrusions according to the disclosure,

Figs. 17A-17B illustrates a schematic example of a plurality of protrusions according to the disclosure,

Fig. 18 illustrates a perspective view of a tubular construct having one or more apertures

Fig. 19 illustrates tested tubular construct geometries according to the disclosure,

Fig. 20 illustrates testing results for polymer stress-strain curve,

Fig. 21 illustrates testing results for polymer stress-strain curve,

Fig. 22A-22C illustrate components of a designed test urethra,

Fig. 23 illustrates a completed designed test urethra,

Fig. 24 illustrates tensile strength results of the tested geometries of Fig. 19,

Fig. 25 shows images of a bladder after the extraction and the consequent histopathological analysis of control vs treatment after 10 and 30 days. Images at 10x and 40x,

Fig. 26 shows images of the co-polymer films inserted into a rat bladder at 0, 10, 20, and 30 days.

## DETAILED DESCRIPTION

The present invention is based on a biodegradable material prepared from monomers present in nature, said monomers having been provided from a natural or synthetic source. More particularly, the invention is based on co-polymerization of two monomers. The co-polymers as defined herein may be used to form tubular constructs, such as tubular stents, which are useful for insertion into a body lumen, such as the urethra. Once inserted, the tubular constructs avoid obstruction of the body lumen in a manner known in the art for e.g. stents. Due to the particular properties of the co-polymers, the tubular construct will be absorbed or excreted by the body at a rate allowing it to fulfil its function for the desired time, while avoiding the need for subsequent removal by surgery.

Accordingly, in a first aspect, the present invention concerns a tubular construct suitable for insertion into a body lumen, such as the urethra, said construct comprising a polymeric material prepared by co-polymerization of ε-caprolactone, δ-valerolactone, ethylene brassylate, or δ-hexalactone with lactide. In a further aspect, the present invention concerns a polymeric material prepared by co-polymerization of ε-caprolactone, δ-valerolactone, ethylene brassylate, or δ-hexalactone with lactide for use in the prevention of obstruction of a body lumen, such as the urethra.

### *CO-POLYMERS*

The tubular construct according to the invention comprises a polymeric material prepared by co-polymerization of ε-caprolactone, δ-valerolactone, ethylene brassylate, or δ-hexalactone with lactide. Lactide is the cyclic lactone ester resulting from esterification between two lactic acid molecules. Several stereoisomeric forms of lactide exist, including L-lactide, D-lactide, and meso-lactide. Furthermore, racemic lactide is a 50:50 mixture of L-lactide and D-lactide. Thus, in one embodiment, the lactide is selected from L-lactide, D-lactide, meso-lactide, racemic lactide, as well as mixtures thereof. In a further embodiment, the lactide is racemic lactide.

The monomer to be co-polymerized with lactide is selected from the group consisting of ε-caprolactone, ε-decalactone, δ-valerolactone, ethylene brassylate, and δ-hexalactone. In one embodiment, the monomer to be co-polymerized with lactide is selected from the group consisting of ε-caprolactone, δ-valerolactone, ethylene brassylate, and δ-hexalactone. In a further embodiment, the monomer to be co-polymerized with lactide is selected from the group consisting of ε-caprolactone, δ-valerolactone, and ethylene brassylate. In still a further embodiment, the monomer to be co-polymerized with lactide is selected from the group consisting of ε-caprolactone and δ-valerolactone. In yet a further embodiment the monomer to be co-polymerized with lactide is ε-caprolactone.

Depending on the specific needs for e.g. time of absorption of the polymeric material by the body or particular mechanical properties, the molecular weight of the polymeric material may be adjusted. This can *inter alia* be achieved by adjusting the polymerization conditions, such as time, temperature, catalyst, and stirring rate. The molecular weight may be indicated as the number average molecular weight or the weight average molecular weight. In one embodiment, the number average molecular weight of the polymeric material is in the range 15 kDa to 300 kDa. In another embodiment, the number average molecular weight of the polymeric material is in the range 35 kDa to 125 kDa. In still another embodiment, the number average molecular weight of the polymeric material is in the range 45 kDa to 75 kDa. In a further embodiment, the weight average molecular weight of the polymeric material is in the range 30 kDa to 600 kDa. In yet a further embodiment, the weight average molecular weight of the polymeric material is in the range 70 kDa to 250 kDa. In still a further embodiment, the weight average molecular weight of the polymeric material is in the range 90 kDa to 150 kDa.

The molecular weight (weight average as well number average) of the polymeric material may be determined by Gel Permeation Chromatography-Size Exclusion Chromatography (GPC-SEC) with polystyrene standards (as specified in Fernández, J. et al, Journal of the Mechanical Behavior of Biomedical materials, 64, 2016, 209-219) as well as the dispersity (=$M_w/M_n$).

The proportion of the monomeric materials used for the polymerization may be adjusted as needed for the optimal result. Typically, the amount of lactide is higher than the amount of ε-caprolactone, δ-valerolactone, ethylene brassylate, or δ-hexalactone. In one embodiment, the molar fraction of ε-caprolactone, δ-valerolactone, ethylene brassylate, or δ-hexalactone is in the range between 2 to 35 of the polymeric material and the molar fraction of lactide is in the range between 98 to 65 of the polymeric material. In a further embodiment, the molar fraction of ε-caprolactone, δ-valerolactone,

ethylene brassylate, or δ-hexalactone is in the range between 8 to 20 of the polymeric material and the molar fraction of lactide is in the range between 92 to 80 of the polymeric material. In still a further embodiment, the molar fraction of ε-caprolactone, δ-valerolactone, ethylene brassylate, or δ-hexalactone is in the range 8 to 18 of the polymeric material and the molar fraction of lactide is in the range 92 to 82 of the polymeric material.

*PREPARATION OF POLYMERS*

A procedure for obtaining co-polymers (or ter-polymers) with mainly random arrangement based on the stereoisomers of the lactones derived from (*S*)-Lactic acid and (*R*)-Lactic acid comprises a single synthetic step using a bulk polymerization method:

Monomers used:

a) Main monomer: Lactones derived from (*S*)-Lactic acid and (*R*)-Lactic acid (lactides)

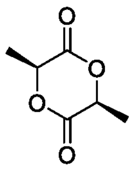

(3*S*,6*S*)-3,6-dimethyl-1,4-dioxane-2,5-dione
(3*S*,6*S*)-Lactide
L-Lactide
CAS Number: 4511-42-6

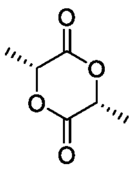

(3*R*,6*R*)-3,6-dimethyl-1,4-dioxane-2,5-dione
(3*R*, 6*R*)-Lactide
D-Lactide
CAS Number: 13076-17-0

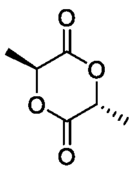

(3*R*,6*S*)-3,6-dimethyl-1,4-dioxane-2,5-dione
trans-3,6-dimethyl-1,4-dioxane-2,5-dione
(3*R*,6*S*)-Lactide
(3*S*,6*R*)-Lactide
*meso*-Lactide
CAS Number: 13076-19-2

**Racemic mixtures:**

50/50 enantiomer (*R*) or D-Lactide and enantiomer (*S*) or L-Lactide

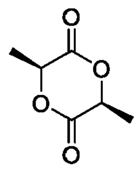

+

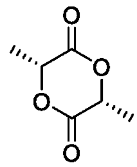

50% 50%

(D,L)-Lactide
*rac*-Lactide
(±)-Lactide
CAS Number: 95-96-5

b) Secondary monomer: Other cyclic esters (Lactones)

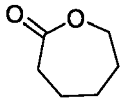

ε–caprolactone

CAS Number: 502-44-3

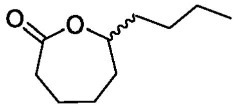

ε–decalactone
(Racemic)

CAS Number: 5579-78-2

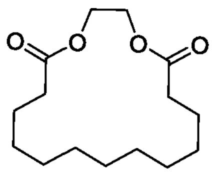

Ethylenbrassylate

CAS Number: 105-95-3

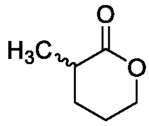

δ-hexalactone
(Racemic)

CAS Number: 823-22-3

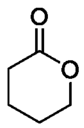

δ-valerolactone

Cas Number:542-28-9

For these polymers it can be considered that they are random co-polymers, if their degree of randomness (R) is between $0.80 \leq R \leq 1.20$. The reaction may be carried out in a single synthetic reaction step, regardless of the order of addition of the different monomers and the catalyst.

The reaction is classified as a ROP (*Ring Opening Polymerization*) type reaction, based on the release of energy from the lactone rings, through a catalyst or initiator. These ROP catalysts or initiators may be:

a. Metal Catalyst (or metalloid), with a metal or metalloid as active center: Metal salts, Organometallic compounds, regardless of the oxidation state of the active center and their non-metallic counterparts. Including metals and metalloids such as Sn, Y, Bi, Al, Cu, Zn, Sb, Fe, Ni or Co

b. Organocatalyst: Such as nucleophilic nitrogenated bases (1,8-Diazabicyclo[5.4.0]undec-7-ene (DBU), 4-Dimethylaminopyridine (DMAP), 7-Methyl-1,5,7-triazabicyclo[4.4.0]dec-5-ene (MTBD), 1,5,7-Triazabicyclo[4.4.0]dec-5-ene (TBD)). Thiourea derivatives, sulfonamides phosphines, phosphazene-based bases and N-heterocyclic carbenes, Acidic catalyst (diphenylphosphate (DPP), triflic acid (TfOH), Methanesulfonic acid (MsOH), Phosphoramidic acid.

The total sum of the main monomer and the secondary monomer preferably on the reaction maintains a molar ratio with the catalyst/initiator, between 100:1 and 10,000:1, depending temperature and the intrinsic reactivity of the secondary monomer. The final molar composition for the polymers synthesized in this invention is preferably between 2% and 35% with refence to the secondary monomer, regardless of the composition of the initial feed mixture. The final molar composition for the polymers synthesized in this invention is preferably between 65% and 98% with reference to the main monomer, regardless of the composition of the initial feed mixture.

The reaction time can range from 1 hour to 72 hours (3 days) of reaction depending on the intrinsic reactivity of the secondary monomer, the activity or nature of the catalyst, concentration of catalyst and the reaction temperature. -The reactions may be carried out in an inert atmosphere of Argon (Ar) or Nitrogen ($N_2$). However, there are some catalysts that do not lose catalytic activity, even in the presence of atmospheric oxygen (e.g Triphenylbismuth ($BiPh_3$) or (1,8-Diazabicyclo[5.4.0]undec-7-ene (DBU)). The method has the advantage of avoiding the use of stannous octoate, a catalyst often used in polymerization reactions, and thus avoiding trace amounts of this potentially cytotoxic substance coming into contact with body tissue. Thus, in one embodiment, the polymeric material of the invention is prepared using a catalyst using substantially no stannous octoate. In a further embodiment, no stannous octoate is used in the preparation

of the polymeric material. In still a further embodiment, the tubular constructs of the invention do not contain any detectable amounts of stannous octoate.

*MEDICAL USES*

The tubular constructs of the invention are suitable for use in preventing obstruction of body lumens. Such lumens include, but are not limited to, the urethra, the ureter, blood vessels, ducts of the digestive system, the pharynx, the esophagus, the small intestine, the large intestine, the biliary system, the bile duct, the cystic duct, the pancreatic duct, the respiratory tract, the larynx, the windpipe, and the bronchioles, in particular the urethra. Stenosis of body lumens, such as the urethra, may have congenital causes or be caused by trauma, infections, or be a side effect of a surgical procedure. The stenosis is typically removed surgically and a tubular construct, such as a stent, may be used to maintain the lumen open for passage of the physiological material that is transported through the lumen under normal circumstances. Thus, in a further aspect, the present invention concerns a polymeric material prepared by co-polymerization of ε-caprolactone, δ-valerolactone, ethylene brassylate, or δ-hexalactone with lactide for use in the prevention of obstruction of a body lumen, such as the urethra. In one embodiment, the polymeric material is used in the prevention of obstruction of a body lumen, such as the urethra, after surgical removal of stenosis. In a further embodiment, the invention concerns the polymeric material of the invention for use in the prevention of obstruction of the urethra after surgical removal of stenosis. In yet a further embodiment, the duration of the use is about 2 to 90 days, such as about 5 to 45 days, for example about 6 to 35 days or about 7 to 30 days. Without being bound by a particular theory, a rigid, continuous tubular construct of the invention may be beneficial to protect the zone of the urethra that has been treated during the previous stenosis surgery by allowing the urethra to epithelise from its healthy edges. Moreover, differently to mesh-like, expansible tubular constructs, such rigid, continuous tubular constructs may protect the damaged zone from being in contact with urine, thus diminishing the risk of infections.

*STENT DESIGN*

Disclosed herein are one or more example tubular constructs. The tubular constructs may be, for example, one or more of: tubes, devices, implants, surgical implants, cylinders, medical devices, tubular devices, stents, spiral-shape stents, and tubular stents. The tubular stent may be formed from the material discussed above. The tubular construct may be completely formed from the material discussed above. The tubular construct is rigid.

The tubular construct includes a radially outward surface. The tubular construct includes a radially inward surface. The radially inward surface may be opposite from the radially outward surface. The tubular construct can include a first end and a second end. The first end and the second end may be considered longitudinal ends of the tubular construct. As used herein, "surface" denotes an outer wall of the tubular construct. Accordingly, the tubular construct is delimited by the radially inward surface (which is its innermost wall) and the radially outward surface (which is its outermost wall). In preferred examples, the radially inward surface and the radially outward surface are continuous walls. As used herein, the terms "continuous wall" or "continuous surfaces" refer to walls or surfaces that present no discontinuities along its entire longitudinal dimension or along a significant part (such as at least 80%, preferably 90%, more preferably 95%) of its longitudinal dimension.

The tubular construct is hollow and may be cylindrical, or generally cylindrical shape. The particular shape is not limiting, and modifications to the tubular construct may be used. The tubular construct may have a longitudinal centerline (e.g., longitudinal axis) extending through the tubular construct. The longitudinal centerline may extend from the first end to the second end of the tubular construct. The tubular construct may have a longitudinal length. For example, the tubular construct can have a longitudinal length of 5, 10, 15, 20, 25, 30, 35, 40, 45, 50, 55, 60, 65, 70, 75, 80, 85, or 90mm. The tubular construct can have a longitudinal length of greater than 5, 10, 15, 20, 25, 30, 35, 40, 45, 50, 55, 60, 65, 70, 75, 80, 85, or 90mm. The tubular construct can have a longitudinal length of less than 5, 10, 15, 20, 25, 30, 35, 40, 45, 50, 55, 60, 65, 70, 75, 80, 85, or 90mm.

The tubular construct may have an outer diameter of 3, 4, 5, 6, 7, 8, 9, or 10mm. The tubular construct may have an inner diameter of 3, 4, 5, 6, 7, 8, 9, or 10mm.

The tubular construct may have a thickness (e.g., from and/or between the radially inward surface to the radially outward surface) of 0.1, 0.2, 0.3, 0.333, 0.4, 0.5, 0.6, 0.7, or 0.8mm. The tubular construct may have a thickness of greater than 0.1, 0.2, 0.3, 0.333, 0.4, 0.5, 0.6, 0.7, or 0.8mm. The tubular construct may have a thickness of less than 0.1, 0.2, 0.3, 0.333, 0.4, 0.5, 0.6, 0.7, or 0.8mm. The thickness of the tubular construct may vary. For example, the thickness of the tubular construct may vary along the longitudinal length of the tubular construct. The thickness of the tubular construct may not vary.

The tubular construct includes a lumen. The lumen may be defined (e.g., formed, bound) by the radially inward surface of the tubular construct. The longitudinal centerline may extend though the lumen. The lumen may have a circular cross section. The lumen may have an ovaloid cross section. The lumen may have a polygonal cross section.

The lumen may extend though the tubular construct. The lumen may extend along a longitudinal axis of the tubular construct. Fluids, and other particular smaller than the lumen, may pass through the lumen from one end of the tubular construct to an opposite end of the tubular construct.

The lumen may have the same dimensions throughout the tubular construct. For example, the lumen can be generally cylindrical in shape. The lumen may vary in dimensions through the tubular construct. For example, the radially inward surface may move towards or away from the longitudinal centerline.

In one or more example tubular constructs, the tubular construct may include one or more protrusions. In one or more example tubular constructs, the tubular construct may include a plurality of protrusions. The plurality of protrusions may be on (e.g., extend from) the radially outward surface of the tubular construct. The plurality of protrusions may be on the radially inward surface of the tubular construct. The plurality of protrusions may not be on the radially inward surface of the tubular construct. The plurality of protrusions may be integrally formed with the tubular construct. The plurality of protrusions may be attached to the tubular construct, such as on the radially outward surface of the tubular construct. For example, the plurality of protrusions may be mechanically or chemically attached to the tubular construct.

The plurality of protrusions may have a longitudinal length of 0.5, 1.0, 1.5, 2.0, 2.5, 3.0, or 3.5 mm. The particular longitudinal length is not limiting. The plurality of protrusions may extend away from the radially outward surface 0.1, 0.2, 0.3, 0.4, 0.5, 0.6, 0.7, 0.8, 0.9, 1.0, 1.1, 1.2, 1.3, 1.4, or 1.5mm.

In one or more example tubular constructs, the tubular construct may include a single protrusion. For example, the single protrusion can be formed from a plurality of interconnected protrusions, as discussed below. Accordingly, connected protrusions as discussed herein may be understood as a single protrusion.

The plurality of protrusions can be configured to provide a radially outward force. For example, the plurality of protrusions can provide a frictional force on a surface radially outwards from the tubular construct. For example, the plurality of protrusions can be configured to provide a frictional force on tissue in contact with the radially outward surface. This may advantageously help keep the tubular construct in place once implanted (e.g., prevent non-desired movement of the tubular construct).

In one or more example tubular constructs, the plurality of protrusions can be one or more of: knobs, rings, helixes, spirals, extensions, bubbles, protrusions, humps, protuberances, bumps, nubs, and any irregular pattern of protuberances. The plurality of protrusions can be corrugation. The plurality of protrusions can create a corrugated structure on the radially outward surface of the tubular construct. The plurality of protrusions can be frictionally increasing protrusions. The plurality of protrusions can extensions. The plurality of protrusions can be rings. The plurality of protrusions can be a textured surface.

The plurality of rings can be perpendicular to the lumen and/or the longitudinal centerline. The plurality of rings can be parallel to the lumen and/or the longitudinal centerline. The plurality of rings can be angled with respect to the lumen and/or the longitudinal centerline.

In one or more example tubular constructs, the plurality of protrusions can be a plurality of circumferential rings. Each of the plurality of circumferential rings may extend partially or fully around the circumference of the tubular construct. Adjacent rings of the plurality of circumferential rings can be connected. Adjacent rings of the plurality of circumferential rings can be disconnected. For example, adjacent rings of the plurality of circumferential rings can be longitudinally spaced apart by 0.5, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, or 10.0mm, though the particular dimensions are not limiting.

The plurality of circumferential rings can be perpendicular to the lumen and/or the longitudinal centerline. The plurality of circumferential rings can be parallel to the lumen and/or the longitudinal centerline. The plurality of circumferential rings can be angled with respect to the lumen and/or the longitudinal centerline.

In one or more example tubular constructs, each of the plurality of circumferential rings can be the same angle with respect to the lumen and/or the longitudinal centerline. In one or more example tubular constructs, one or more of the plurality of circumferential rings can be at a different angle with respect to the lumen and/or the longitudinal centerline from another one of the plurality of circumferential rings. In one or more example tubular constructs, each of the plurality of circumferential rings can be randomly angled with respect to the lumen and/or the longitudinal centerline.

As mentioned, the plurality of protrusions, such as the plurality of circumferential rings, extend radially away from the radially outward surface of the tubular construct.

In one or more example tubular constructs, each of the plurality of circumferential rings can be rounded. In one or more example tubular constructs, at least one of the plurality of circumferential rings can be rounded. In one or more example tubular constructs, each of the plurality of circumferential rings can include at least one rounded edge. In one or more example tubular constructs, at least one of the plurality of circumferential rings can include at least one rounded edge.

In one or more example tubular constructs, each of the plurality of circumferential rings can include at least one edge. In one or more example tubular constructs, at least one of the plurality of circumferential rings can include at least one edge. The at least one edge can be a non-rounded edge.

In one or more example tubular constructs, at least one of the plurality of circumferential rings can include at least one edge and at least one of the plurality of circumferential rings can be rounded. In one or more example tubular

constructs, one or more of the plurality of circumferential rings can have a rounded edge and a non-rounded edge.

In one or more example tubular constructs, the plurality of circumferential rings may be angled towards the first end or the second end of the tubular construct. Alternatively, the plurality of circumferential rings may be centered (e.g., not angled towards a particular end of the tubular construct).

The plurality of protrusions, such as the plurality of circumferential rings, may be one or more of: square shaped, rectangular shaped, triangular shaped, circular shaped, ovaloid shaped, semi-circle shaped, trapezoidal shaped, curved shaped, quarter-circle shaped, polygonal shaped, and any irregular pattern. For example, the plurality of protrusions may be triangularly shaped, and thus may have one edge not directly integrated with the tubular construct (e.g. one free edge). If the plurality of protrusions are square shaped, they may have two edges not directly integrated with the tubular construct (e.g., two free edges). If the plurality of protrusions are circular or ovaloid shaped (or curved), they may not have a free edge. In one or more example tubular constructs, the plurality of protrusions may have a radius of curvature of 0.1, 0.2, 0.3, 0.4, 0.5, 0.5, 0.7, 0.8, or 0.9mm.

In one or more example tubular constructs, adjacent ones of the plurality of circumferential rings can be disconnected. In one or more example tubular constructs, all the plurality of circumferential rings can be disconnected from one another. For example, each of the plurality of circumferential rings is spaced longitudinally apart from adjacent ones of the plurality of circumferential rings. There can be a gap between adjacent ones of the plurality of circumferential rings.

In one or more example tubular constructs, adjacent ones of the plurality of circumferential rings can be connected. In one or more example tubular constructs, all the plurality of circumferential rings can be connected. For example, the plurality of circumferential rings can be connected together to form a helical shape along the radially outward surface of the tubular construct. In one or more example tubular constructs, the plurality of protrusions, such as the plurality of circumferential rings, can form a helix.

In one or more example tubular constructs, some adjacent ones of the plurality of circumferential rings can be connected while other adjacent ones of the plurality of circumferential rings can be disconnected.

In one or more example tubular constructs, the plurality of protrusions, such as the plurality of circumferential rings, can extend to the longitudinal ends of the tubular construct. For example, a circumferential ring may make up a first and/or a second end of the tubular construct.

Alternatively, the plurality of protrusions, such as the plurality of circumferential rings, may not extend to the longitudinal ends of the tubular construct. Thus, there may be a gap between the first and/or second end of the tubular construct and the first of the plurality of protrusions, such as the plurality of circumferential rings. For example, the plurality of circumferential rings can be spaced longitudinally apart from longitudinal ends of the tubular construct.

For example, there may be a gap from the first end to the nearest of the plurality of protrusions of 0.0, 0.5, 1.0, 1.5, 2.0, 2.5, 3.0, 3.5, or 4.0mm.

In one or more example tubular constructs, the plurality of protrusions, such as the plurality of circumferential rings, can extend to the first end of the tubular construct but may not extend to the second end of the tubular construct.

In one or more example tubular constructs, the tubular construct can further include one or more apertures (e.g. lumens, gaps, perforations, openings, holes). The one or more apertures can extend from the radially outward surface of the tubular construct to the radially inward surface of the tubular construct. The particular shape and size of the one or more apertures is not limiting.

Advantageously, the one or more apertures can allow fluid to extend from the lumen of the tubular construct to outside of the tubular construct. For example, the one or more apertures can allow for the drainage of pus from a possible infection in a wound or sore. Moreover, the one or more apertures can further improve frictional adhesion to tissue.

The one or more apertures can extend through one or more of the plurality of protrusions, such as the plurality of circumferential rings. The one or more apertures may not extend through any of the plurality of protrusions.

The one or more apertures may be spaced equally around a circumference of the tubular construct. The one or more apertures may not be spaced equally around a circumference of the tubular construct.

The one or more apertures may be spaced equally around a longitudinal length of the tubular construct. The one or more apertures may not be spaced equally around the longitudinal length of the tubular construct.

## *DETAILED DESCRIPTION OF THE FIGURES*

FIGS. 1A-1B illustrate a perspective view (1A) and longitudinal view (1B) of a tubular construct 50 without any protrusions, also known as a smooth tube. While this design may be easy to insert, such as into a patient, it does not have any external shaping, such as the plurality of protrusions, and thus will assert minimal frictional forces. Therefore, the risk of axial displacement will be high.

FIGS. 2A-2C illustrate an example tubular construct 100 according to the disclosure. Fig. 2A illustrates a perspective view, Fig. 2B illustrates a cross section down a longitudinal length, and Fig. 2C illustrates a radial cross-section. The hatched area indicates a thickness of the tubular construct 100. The outer ring indicates height, e.g. radial

length, of the plurality of protrusions 200. The tubular construct 100 can have a first end 102, a second end 104, and a body 106 extending between the first end 102 and the second end 104. Further, the tubular construct 100 can include a radially outward surface 108 and a radially inward surface 110. The radially inward surface 110 of the body 106 can define (e.g. form) a lumen 112 passing through a longitudinal length of the tubular construct 100 along a longitudinal axis 114 (shown in Fig. 2B). The lumen 112 can extend from the first end 102 to the second end 104, thus providing for openings in the first end 102 and the second end 104 for fluid to pass through.

As shown, the tubular construct 100 can include a plurality of protrusions 200. In this figure, the plurality of protrusions 200 are a plurality of circumferential rings 202. As discussed in detail throughout, the plurality of protrusions 200 can include variations. Advantageously, the plurality of protrusions 200 can provide a frictional force when the tubular construct 100 is implanted.

The plurality of circumferential rings 202 are aligned to be perpendicular to the longitudinal axis 114 and/or the lumen 112. Thus, the plurality of circumferential rings 202 can extend circumferentially around the body 106 of the tubular construct 100 on the radially outward surface 108. As shown, the plurality of circumferential rings 202 (e.g., the plurality of protrusions 200) can be integrally formed with the body 106. Alternatively, the plurality of circumferential rings 202 (e.g., the plurality of protrusions 200) could be attached to the body 106 of the tubular construct 106.

The plurality of circumferential rings 202 can be rounded (e.g. curved, spherical, ovaloid), as shown in the figures. Thus, the plurality of circumferential rings 202 may not include an edge. In the tubular construct 100 shown, the circumferential rings 202 may appear as half-circles in cross section.

Further, as shown each of the plurality of circumferential rings 202 can be longitudinally spaced away (e.g., separated, spaced, disconnected) from adjacent ones of the plurality of circumferential rings 202. Further, the plurality of circumferential rings 202 can be spaced longitudinally spaced away (e.g., separated, spaced, disconnected) from the first end 102 and the second end 104.

Figs. 3A-4B illustrate a tubular construct 100 having a triangular shaped plurality of protrusions 200A/200B. Accordingly, the plurality of protrusions 200A/200B can be formed from a first wall and a second wall with a corner between the first wall and the second wall.

Specifically, Figs. 3A-3B show a plurality of protrusions 200A being right triangles. Advantageously, the right triangles can facilitate insertion into the penis, and minimize pain on the part of the patient. Furthermore, the plurality of protrusions 200A could insert into the mucosa minimizing axial movement during urination.

Figs. 4A-4B show a plurality of protrusions 200B being equilateral triangles. Similar to Figs. 3A-3B, the plurality of protrusions 200B can facilitate insertion into the penis. By being symmetric in geometry, this design can also be inserted in both directions (e.g., in either direction).

Figs. 5A-5B illustrate a tubular construct 100 having a rectangular shaped plurality of protrusions 200C. Accordingly, the plurality of protrusions 200C can be formed from a first wall, a top wall, and a second wall with a first corner between the first wall and the top wall, and a second corner between the top wall and the second wall. The plurality of protrusions can vary in dimensions (such as width and/or height) along the longitudinal length of the tubular construct. Advantageously, the design can offer a fairly high friction force on both sides, which can prevent the tube from moving both outward and inward. Further, the design can be inserted in both directions.

Figs. 6A-8B illustrate a tubular construct 100 having curved and/or circular shaped plurality of protrusions 200D/200E/200F.

Figs. 6A-6B illustrate a plurality of protrusions 200D having a semicircular shape. This design can be inserted in both directions. Further, the plurality of protrusions 200D can provide sufficient frictional force to prevent the tubular construct 100 from moving. Moreover, the plurality of protrusions 200D can allow easy insertion into the urethra since it has curved shapes without any spike that may be painful for the patient.

Figs. 7A-7B illustrate a plurality of protrusions 200E having a quarter-circle shape. This design can advantageously allow for simple insertion, as well as providing a strong frictional force to prevent movement due to the perpendicular wall. Moreover, its pain can be minimized during insertion due to the curved shape which allows an ease of movement. That being said, the vertical walls will allow us to create a greater friction force to prevent the tubular construction 100 from coming out through the urethra.

Figs. 8A-8B illustrate a plurality of protrusions 200F having a curved shape. The plurality of protrusions 200F can be configured to engage mucosa, creating frictional forces that will prevent the tubular construct 100 from moving. Moreover, without the use of any sharp corners and/or peaks, it can be easily inserted into a patient from either direction.

Figs. 9A-12 illustrate a tubular construct 100 having different combined shapes for the plurality of protrusions 200G/200H/200I/200J.

Figs. 9A-9B illustrate a plurality of protrusions 200G having a half circle shape combined with a rectangular shape. This design allows can allow for easy insertion through the curved part on one side of the tubular construct 100. Further, it can have an excellent grip since it has vertical walls which will create a great grip force within the urethra. The rectangular portion of the plurality of protrusions 200G can vary in width and the number of geometries can be variable.

Figs. 10A-10B illustrate a plurality of protrusions 200H having a right triangle shape combined with a rectangular

shape. This design allows easy insertion. Further, it has a good grip since it has vertical walls which will present a great friction force so that the tube does not move inside the urethra. The rectangular part can vary in width and the number of geometries can be variable.

Figs. 11A-11B illustrate a plurality of protrusions 2001 having a quarter circle shape, a rectangle shape, and another quarter circle shape. The rectangular part can lengthen the geometry of the half circle. It allows inserting it from both sides, being painless and easy to handle. Moreover, it would create the necessary friction force so that the non-movement of the tutor tube is not allowed. The number of geometries and the width of the rectangular part are variable.

Fig. 12 illustrate a plurality of protrusions 200J having a triangular shape, a rectangle shape, and another triangular shape. The rectangular part can lengthen the geometry of the triangular shapes. It allows inserting it from both sides, being painless and easy to handle. Moreover, it would create the necessary friction force so that the non-movement of the tutor tube is not allowed. The number of geometries and the width of the rectangular part are variable.

Figs. 13A-17B illustrate a tubular construct 100 having asymmetric protrusions 200K/200L/200M/200N/200O. In particular, the plurality of protrusions can all be connected and rotate around a circumference of the tubular construct 100. Alternatively, there may be connections or gaps between adjacent protrusions of the plurality of protrusions. For example, they may spiral around the tubular construct 100, forming a helix/helical shape.

Figs. 13A-14B illustrate a tubular construct 100 having helical protrusions 200K/200L.

Advantageously, depending on the shape of the plurality of protrusions and the pitch of the plurality of protrusions along the tubular construct 100, different geometries can be obtained. As shown in Fig. 13A-13B, the plurality of protrusions 200K may have a right-triangular shape, whereas Figs. 14A-14B shows a plurality of protrusions 200L which may have an equilateral triangle shape.

Figs. 15A-17B illustrate a tubular construct 100 having double helical protrusions 200M/200N/200O. In particular, one set of the plurality of protrusions can all be connected and rotate around a circumference of the tubular construct 100. A second set of the plurality of protrusions can be connected and rotate in the opposite direction. For example, both sets of the plurality of protrusions may spiral around the tubular construct 100, forming a helix/helical shape.

Figs. 15A-15B illustrate a tubular construct 100 with two sets of a plurality of protrusions 200M forming a double helix shape on the radially outward surface 108 of the tubular construct 100.

Advantageously, depending on the shape of the plurality of protrusions and the pitch of the plurality of protrusions along the tubular construct 100, different geometries can be obtained. As shown in Figs. 16A-16B, the plurality of protrusions 200N may have a right-triangular shape, whereas Fig. 17A-17B show a plurality of protrusions 200O which may have an equilateral triangle shape.

Fig. 18 illustrates a tubular construct 100 having one or more apertures 300. The one or more apertures 300 can extend between the radially outward surface 108 and the radially inward surface 110. While the one or more apertures 300 are shown in a particular tubular construct 100, the one or more apertures 300 can be used in any and/or all of the variations discussed herein.

The use of the terms "first", "second", "third" and "fourth", "primary", "secondary", "tertiary" etc. does not imply any particular order, but are included to identify individual elements. Moreover, the use of the terms "first", "second", "third" and "fourth", "primary", "secondary", "tertiary" etc. does not denote any order or importance, but rather the terms "first", "second", "third" and "fourth", "primary", "secondary", "tertiary" etc. are used to distinguish one element from another. Note that the words "first", "second", "third" and "fourth", "primary", "secondary", "tertiary" etc. are used here and elsewhere for labelling purposes only and are not intended to denote any specific spatial or temporal ordering. Furthermore, the labelling of a first element does not imply the presence of a second element and viceversa.

It is to be noted that the word "comprising" does not necessarily exclude the presence of other elements or steps than those listed.

It is to be noted that the words "a" or "an" preceding an element do not exclude the presence of a plurality of such elements.

It should further be noted that any reference signs do not limit the scope of the claims.

## EXAMPLES

*EXAMPLE 1 - preparation of a co-polymer of ε-caprolactone and racemic lactide*

Synthesis of poly(D,L-lactide-co-ε-caprolactone) (P(DLLA-CL)) co-polymer with a feed composition of 80:20 (% w/w) using Triphenylbismuth ($BiPh_3$) as catalyst was carried out in bulk by one pot one step ring opening polymerization, with a molar ratio of 1500:1 co-monomers:catalyst. Triphenylbismuth was poured into the reactor when the temperature of the bulk reached 130 °C. The reaction mixture was stirred during 3h at 130 °C.

After this time, the bulk reaction was dissolved in dichloromethane and precipitated in an excess of cold methanol. The slurry obtained was dried in a vacuum-oven at 85°C and reduced pressure.

The obtained co-polymer has a final molar composition as measured by $^1H$-NMR spectra of 85/15 D,L-Lactide/ε-caprolactone with a $M_w$ of 133 kDa and a dispersity value of 1.93. Table 1 summarizes the characterization data of the synthesized co-polymer (dispersity or $M_w/M_n$ (D), average length units of lactide and caprolactone ($l_{LA}$ and $l_{CL}$), randomness character (R) and glass transition temperature (Tg))

*Table 1- Characterization data of P(DLLA-co-CL) co-polymer*

| Feed molar composition | Final molar composition | (%w/w) composition | Yield (%) | $M_w$ (KDa) | D | $l_{LA}$ | $l_{CL}$ | R | $T_g$ (°C) |
|---|---|---|---|---|---|---|---|---|---|
| 76/24 | 85/15 | 88/12 | 86 | 133 | 1.61 | 7.14 | 1.21 | 0.96 | 30.1 |

The final co-polymer composition is richer in D,L-lactide co-monomer comparing to the feed due to co-monomer reactivity differences. D,L-lactide is a small ring lactone (6-member ring, higher ring strain) and thus more reactive than ε-caprolactone (7-member ring lactone, lower ring strain), leading to a faster consumption of the more reactive co-monomer. According to thermal properties, amorphous P(DLLA-CL) co-polymer showed a glass transition temperature ($T_g$) of 30.1 °C.

The repeating units of poly(D,L-lactide-co-ε-caprolactone) are:

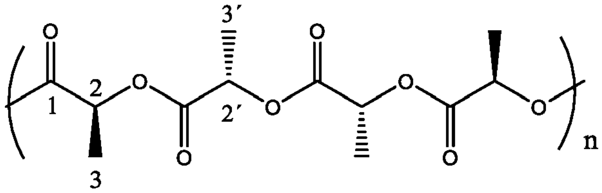

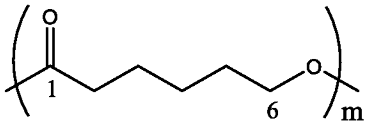

In Table 2 are shown the assignment of different protons and carbons in poly(DL-lactide-co- ε-caprolactone) (as shown above), for the calculation of molar composition and microstructural parameters. Molar composition of the P(DLLA-CL) co-polymer was calculated by averaging the results obtained from $^1$H-NMR spectroscopy.

*Table 1. Chemical shift assignment of protons from a P(DLLA-CL) co-polymer*

| *H* | *H ε-CL δ* **(ppm)** | $H_{LA}$ *δ* **(ppm)** |
|---|---|---|
| 1 | - | - |
| 2,2' | ≈ 2.25 | 5.05 |
| 3, 3' | ≈ 1.50 | 1.50 |
| 4 | ≈ 1.30 | |
| 5 | ≈ 1.15 | |
| 6 | ≈ 4.05 | |

The chemical shift (δ) depends on the neighboring co-monomers. In the groups with two co-monomers, it can show a different magnetic shielding, due to different arrangement in the polymer sequence. In the case of the co-polymer P(DLLA-ε-CL), we have three different sequences: a) LA-LA, b) CL-LA = c) LA-CL and d) CL-CL

a) 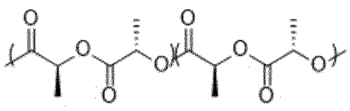 b) 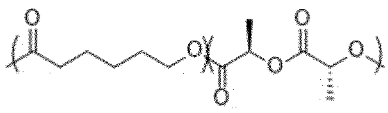

c) 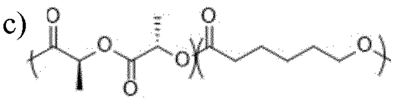

d) 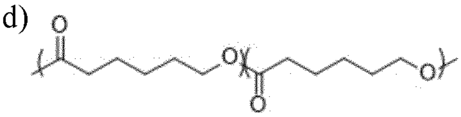

*Composition calculation from the areas in $^1$H-NMR spectra*

As an example of calculation of co-polymer composition based on chemical shift and integrals, we used a co-polymer of P(DLLA-ε-CL) with a feed of 80:20 by weight, whose $^1$H-NMR chemical shifts are shown in Table 3.

Table 3: Assignment table for protons in Poly(D,L-Lactide-co-ε-caprolactone).

| **Signal** | **Integral** | **δ (ppm)** | **H** |
|---|---|---|---|
| A | 1.000 | 5.19 | $2_{(LA)}$ |
| B | 0.139 | 4.14 | $6_{(CL)}$ |
| | 0.026 | 4.08 | |
| C | 0.137 | 2.40 | $2_{(CL)}$ |
| | 0.032 | 2.30 | |

There are two independent signals for each monomer: the A signal only corresponds to protons of the LA monomer (with two -CH- diastereotopic) and the BB and CC signals only contain protons of the ε-CL monomer (the -$CH_2$- in position 6 and the - $CH_2$- in position 2). The rest of the protons are included in the signals between 1.7 and 1.3 ppm.

Therefore, the A, B-B and C-C signals are used to calculate the proton contribution of each co-monomer: the A signal for LA, and the B-B and C-C signals for the ε-CL, using Equation 1

$$(PCH)_{LA} = \frac{A_A}{nº\ H_A} = \frac{1}{2} = 0.5$$

$$(PCH)_{CL} = \frac{A_{B+B+C+C}}{nº\ H_{B+B+C+C}} = \frac{0.575}{4} = 0.14375$$

With the Proton Contribution (CPH), it is possible to calculate the final molar fraction in the polymer of each co-monomer using the equations below:

$$\%\,molar_{LA} = \frac{0.5}{0.5 + 0.14375} \cdot 100 = 77.7$$

$$\%\,molar_{CL} = \frac{0.14375}{0.14375 + 0.5} \cdot 100 = 22.3$$

Taking into account that the molecular weight of LA is 144.13 g/mol and that of ε-CL is 114.14 g/mol, the final composition of the co-polymer by weight is calculated with the equations below:

$$\%\,weight_{LA} = \frac{(0.5 \cdot 144.13)}{(0.5 \cdot 144.13) + (0.14375 \cdot 114.14)} \cdot 100 = 81.5$$

$$\%\,weight_{CL} = \frac{(0.14375 \cdot 144.14)}{(0.14375 \cdot 114.14) + (0.5 \cdot 144.13)} * 100 = 18.5$$

Therefore, from the synthesis of an 80:20 weight feed LA:ε-CL co-polymer, the final weight composition of 81.5: 18.5 was obtained.

*EXAMPLE 2 - preparation of a co-polymer of ethylene brassylate and racemic lactide*

Synthesis of poly(D,L-lactide-co-ethylene brassylate) (P(DLLA-EB)) co-polymer with a feed composition of 70:30 (% w/w) using Triphenylbismuth ($BiPh_3$) as catalyst was carried out in bulk by one pot one step ring opening polymerization, with a molar ratio 100:1 of co-monomers:catalyst. Triphenylbismuth was poured into the reactor when the temperature of the bulk reached 140 °C. The reaction mixture was stirred during 72h at 140 °C.

After this time, the bulk reaction was dissolved in dichloromethane and precipitated in an excess of cold methanol. The slurry obtained was dried in a vacuum-oven at 85°C and reduced pressure.

The obtained co-polymer has a final molar composition as measured by $^1$H-NMR spectra of 95/5 D,L-Lactide/Ethylene brassylate with a $M_w$ of 111.6 kDa and a dispersity value of 2.01. Table 4 summarizes the characterization data of the synthesized co-polymer (dispersity or $M_w/M_n$ (D), average length units of lactide and ethylene brassylate ($l_{LA}$ and $l_{CL}$), randomness character (R) and glass transition temperature (Tg)).

*Table 4 - Characterization data of P(DLLA-co-EB) co-polymer*

| Feed weight composition | Molar composition | (%w/w) composition | Yield (%) | $M_w$ (KDa) | D | $l_{LA}$ | $l_{EB}$ | R | $T_g$ (°C) |
|---|---|---|---|---|---|---|---|---|---|
| 70/30 | 95/5 | 91/9 | 73.1 | 111.6 | 2.01 | 20.37 | 1.13 | 0.932 | 27.9 |

The final co-polymer composition is richer in DL-lactide co-monomer comparing to feed due to reactivity differences of the co-monomers. DL-lactide is a small ring lactone (6-member ring, higher ring strain) and thus more reactive than ethylene brassylate (17-member ring macrolactone, lower ring strain), leading to a faster consumption of the more reactive co-monomer. Regarding the thermal properties, amorphous P(DLLA-EB) co-polymer showed a glass transition temperature ($T_g$) of 27.9 °C in Differential Scanning Calorimetry (DSC) curves.

The repeating units of poly(D,L-lactide-co-ethylene brassylate) are:

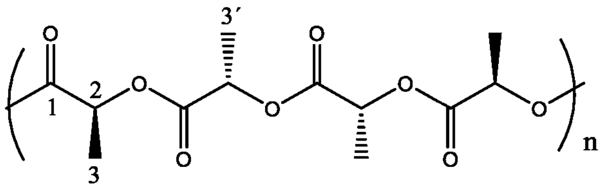

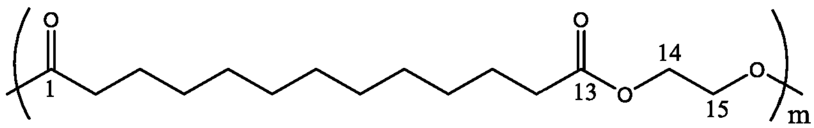

Table 5 shows the assignment of the different protons and carbons in the repeating units poly(DL-lactide-co-ethylene brassylate), for the calculation of molar composition and microstructural parameters. The molar composition of P(DLLA-EB) co-polymer was calculated by averaging the results obtained from $^1$H and $^{13}$ C-NMR spectroscopy.

*Table 5: Chemical shift assignment of protons and carbons from a P(DLLA-EB) co-polymer.*

| C | $C_{EB}$ δ (ppm) | $C_{LA}$ δ (ppm) | H | $H_{EB}$ δ (ppm) | $H_{LA}$ δ (ppm) |
|---|---|---|---|---|---|
| 1 | 172.9-173.6 | 169.9-170.8 LA-EB<br>168.9-169.9 LA-LA | 1 | - | - |
| 2 | 34.0 | 68.6-69.4 LA-LA<br>67.9-68.4 LA-EB | 2 | 2.25 | 5.05 |
| 3 | 24.3-24.5 EB-EB<br>24.6-24.9 LA-EB | 16.72 | 3 | ≈ 1.60 | 1.50 |
| 4 | 29.1-29.5 | | 4 | ≈ 1.30 | |
| 5 | | | 5 | Equal to $H_4$ | |
| 6 | | | 6 | Equal to $H_4$ | |

(continued)

| C | $C_{EB}$ δ (ppm) | $C_{LA}$ δ (ppm) | H | $H_{EB}$ δ (ppm) | $H_{LA}$ δ (ppm) |
|---|---|---|---|---|---|
| 7 | | | 7 | Equal to $H_4$ | |
| 8 | | | 8 | Equal to $H_4$ | |
| 9 | | | 9 | Equal to $H_4$ | |
| 10 | | | 10 | Equal to $H_4$ | |
| 11 | Equal to $C_3$ | | 11 | Equal to $H_3$ | |
| 12 | Equal to $C_2$ | | 12 | Equal to $H_2$ | |
| 13 | Equal to $C_1$ | | 13 | - | |
| 14 | 61.5-63.1 | | 14 | 4.20 | |
| 15 | Equal to $C_{14}$ | | 15 | Equal to $H_{14}$ | |

Table 5 indicates the chemical shifts of the $^1$H-NMR spectrum of the P(DLLA-EB) co-polymer. The analysis was conducted by comparing the signals of the LA methine at 5.05 ppm ($H_2$) with respect to the EB ($-CH_2COO-$ ) methylenes bonded to the ester group, which are centered at 4.20 ppm ($H_{14}$ and $H_{15}$), and EB methylenes bonded to the carbonyl group, centered at 2.25 ppm ($H_2$ and $H_{12}$). $^1$H NMR signals did not show sequence activity and consequently the data of average sequence lengths and randomness character from Table 1 were estimated based on the LA-EB dyad relative molar fractions that were acquired from the $^{13}$C-NMR spectra.

Table 5 also indicates the chemical shifts of the $^{13}$C NMR spectrum of the Poly(DLLA-co-EB) co-polymer with 81% of D,L-lactide content. The molar composition was determined by comparing the areas under the peaks of LA and EB carbons (regardless of EB carbonyl). The average relative value of lactide was calculated using the signals at 170 and 70 ppm ($C_1$ and $C_2$). For ethylene brassylate, the used signals were those at 174 and 25 ppm ($C_1$, $C_3$ and $C_{11}$). Table 2 has also assigned the different dyads from the co-polymer underlying the studied nucleus. The average dyad relative molar fraction (LA-EB) was determined from the estimation of the chain microstructure parameters. This variable is the sum of the (LA-EB) and (LA-EB) average dyad molar fractions and was estimated by multiplying by two the average values of the (LA-EB) and (LA-EB) obtained from LA-EB dyad at 172 and 70 ppm, and the value provided by the LA-EB dyad at 25 ppm.

Bismuth catalysts lead to a random distribution of co-monomers, obtaining a random co-polymer (R=1). Microstructural parameters were calculated using Equations 1-3 the average sequence length of LA ($l_{LA}$) is 4.56 and that of EB ($l_{EB}$) 1.51, obtaining a value randomness R= 0.88.

$$l_A = \frac{(A-A)+\frac{1}{2}(A-B)}{\frac{1}{2}(A-B)} = \frac{2(A)}{(A-B)};\ l_B = \frac{(B-B)+\frac{1}{2}(A-B)}{\frac{1}{2}(A-B)} = \frac{2(B)}{(A-B)} \qquad (1)$$

$$(l_A)_{random} = \frac{1}{(B)};\ (l_B)_{random} = \frac{1}{(A)} \qquad (2)$$

$$R = \frac{(l_A)_{random}}{l_A} = \frac{(l_B)_{random}}{l_{EB}} = \frac{(A-B)}{2(A)(B)} \qquad (3)$$

where (A) and (B) are the molar fractions of co-monomer A and co-monomer B, and (A-A), (A-B) and (B-B) are the average relative molar fractions of dyads A-A, A-B and B-B, respectively (see a) J. Fernández et. al Polymer Degradation and Stability, 2017, 137, 23-34. b) J. Fernández et al. Journal of the Mechanical Behavior of Biomedical Materials, 2012, 9, 100-112. c) E. Prestch, T. Clerc, J. Seibl, W. Simon, Tables of spectral data for structure determination of organic compounds, Chemical Laboratory Practice Book. Springer-Verlag, Berlin Heidelberg, 2013. GmbH.)

*EXAMPLE 3 - Tubular construct geometry*

A number of different tubular constructs geometries were tested with a manufactured urethra. Fig. 19 illustrates the different tested geometries.

Specifically, additive manufacturing (Stratasys Objet260 Connex3™) was used to manufacture the probes and the manufactured urethra. Essentially, this allowed the mixing of two primary materials in different proportions to achieve a

third material with a desired mechanical property. For the experimental design, a mixture of Agilus30 black and a rigid polymer known as Veroblue was used. From these two primary materials, twelve materials were manufactured, six of which have flexible behavior (FLXA) and six of which having rigid behavior RGDA).

1) FLXA-CK-S40-DM

2) FLXA-CK-S50-DM

3) FLXA-CK-S60-DM

4) FLXA-CK-S70-DM

5) FLXA-CK-S85-DM

6) FLXA-CK-S95-DM

7) RGDA-CK-K10-DM

8) RGDA-CK-K20-DM

9) RGDA-CK-K30-DM

10) RGDA-CK-K40-DM

11) RGDA-CK-K50-DM

12) RGDA-CK-K60-DM

Probes were manufactured with the different materials for testing in a tensile test bench to acquire stress-strain curves for specific materials. Geometry was not a factor for this testing as all probes had the same dimensions. As shown in Fig. 20, material K50 can act the most similar to the reference material (Poly(DL-lactide-co-ε-caprolactone) (PLCL) co-polymer).

Further, a mechanical property test based on oral mucosa was performed. In the study Dynamic mechanical properties of oral mucosa: comparison with polymeric soft denture liners. Journal of the Mechanical Behavior of Biomedical Materials, vol. 4 (n° 3). pp. 269-274. ISSN 1751-6161", a comparison between polymers and oral mucosa is performed. It is stated that the Youngs Modulus is about 2.72 MPa.

Fig. 21 illustrates the resulting stress-strain curves, with Table 6 extracting the results. Probes were manufactured with the following materials: FLXA-CK-S60-DM, FLXA-CK-S70-DM, FLXA-CK-S85-DM and FLXA-CK-S95-DM. Accordingly, the most similar material to the known Youngs Modulus is material S85.

*Table 6: Stress-strain material testing results*

| | $\sigma_1$ | $\varepsilon_1$ | $\sigma_2$ | $\varepsilon_2$ | Youngs Modulus (MPa) |
|---|---|---|---|---|---|
| S40 | 0.123 | 0.2 | 0.176 | 0.3 | 0.53 |
| S70 | 0.247 | 0.2 | 0.361 | 0.3 | 1.14 |
| S85 | 0.605 | 0.2 | 0.85 | 0.3 | 2.45 |
| S95 | 0.915 | 0.1 | 1.412 | 0.2 | 4.97 |

Then, a designed test urethra was manufactured in order to test the tubular construct geometries. The designed test urethra is formed from three parts, shown in Figs. 22A-22C: an exterior part (Fig. 22A), an interior part (Fig. 22B), and an auxiliary part (Fig. 22C).

The exterior part shown in Fig. 22A simulates the corpus spongiosum muscle that surrounds the urethra, it is made with the softest material that the 3D printer has, whose Young modulus is 0.45 MPa. The interior part shown in Fig. 22B simulates the urethra itself and it is made with the previously cited material S85. The auxiliary part shown in Fig. 22C is added to the assembly to later be able to carry out the tensile test. By means of this auxiliary part, it was possible to attach the designed test urethra to the jaw of the machine to carry out the tensile tests.

The final construction of the designed test urethra is shown in Fig. 23. Each of the tubular constructs discussed above , with respect to Fig. 19 and made with material K50, were inserted into the interior part without threading to the point where it was not allowed to advance any further by air within the designed test urethra. The tubular construct was then pulled out of the test urethra to determine its "gripping force", such as a frictional force preventing the tubular construct from exiting the designed test urethra. The test was performed to the point where the tubular construct completely exits the hole of the simulated urethra, e.g., the designed test urethra. The test speed was 1 mm / min. Testing results are shown in Table 7. As shown, the tubular construct with the highest "gripping force" is design 5. The tested values come from a 2:1 scale testing, whereas each tubular construct in its insertion state would be at a 1:1 scale.

*Table 7: Urethra testing results*

| GEOMETRY | FORCE IN 2:1 SCALE | FORCE IN 1:1 SCALE (estimated) |
|---|---|---|
| Design 1 | 32.82 N | 8.21 N |
| Design 2 | 21.37 N | 5.34 N |
| Design 3 | 20.35 N | 5.09 N |
| Design 4 | 35.11 N | 8.78 N |
| Design 5 | 49.37 N | 12.34 N |

Further, displacement tests were performed on the tubular constructs shown in Fig. 19 with material K50. A thermal test bench was developed to perform displacement tests when a fluid passes through the tubular constructs in a non-alternating way, that is, the fluid will circulate every " x " time and stop so that the test is as real as possible.

Each tubular construct was tested in two different ways. The first test was carried out with the continuous flow of water for 5 hours and the second one discontinuously for 5 hours. In the case of the discontinuous flow test, it remains active for 30 seconds and off for 5 seconds. Each tubular construct was tested for a whole day, 10 hours in a row without rest, so that the result are as reliable as possible, since, in a real situation, there would be no stoppage. Table 8 illustrates the testing results.

*Table 8: Displacement testing results*

| GEOMETRY | INITIAL POSITION | FINAL POSITION (CONTINOUS) | FINAL POSITION (DISCONTINOUS) |
|---|---|---|---|
| Design 1 | 6,0 mm | 6,0 mm | 6,0 mm |
| Design 2 | 6,0 mm | 6,0 mm | 6,0 mm |
| Design 3 | 6,0 mm | 6,0 mm | 6,0 mm |
| Design 4 | 6,0 mm | 6,0 mm | 6,0 mm |
| Design 5 | 6,0 mm | 6,0 mm | 6,0 mm |

As shown, none of the tubular constructs suffered any measurable displacement, so it can be concluded that any geometry could works in terms of displacement.

*EXAMPLE 4 - preparation of a co-polymer of ε-caprolactone and racemic lactide plus in vivo study*

Poly(DL-lactide-co-ε-caprolactone) (PLCL) co-polymer was synthesized in bulk by a one pot one step ring-opening polymerization (ROP) of each lactone, at 1500:1 co-monomers:catalyst molar ratio. The reaction was carried out over 1 hour in a 2 L reactor vessel, at 130 °C. 500 grams of co-polymer were synthesized with a feed composition of 75:25 (% w/w) ratio lactide: ε-caprolactone. No initiator was added to the reaction mixture and the catalyst ($BiPh_3$) was added when the temperature reached 130°C.

The reaction product was dissolved in dichloromethane and precipitated into an excess of methanol to remove catalyst impurities and those monomers that did not react. The co-polymer was dried at room temperature overnight and a heat treatment was carried out (100 °C under vacuum for 1 hour) to ensure the complete elimination of any remaining solvent. Finally, the product was weighed, and the yield of the synthesis process was obtained.

Co-polymer characterization was conducted studying its composition and microstructure by Proton Nuclear Magnetic Resonance ($^1$H-NMR), thermal properties by Differential Scanning Calorimetry (DSC) and molecular weight distribution by means of Gel Permeation Chromatography (GPC). The obtained results are shown in Table 9:

*Table 9: Characterization data of PLCL co-polymer.*

| Feed weight composition | Final Molar composition | Mw (KDa) | D | $I_{LA}$ | $I_{CL}$ | R | $T_g$ (°C) |
|---|---|---|---|---|---|---|---|
| 75:25 | 83:17 | 138 | 1,82 | 6.25 | 1.25 | 0.96 | 24.6 |

250 μm films were prepared by pressure melting at 150 °C with additional water quenching in a P 200 E (Collin) hot pressing machine. From these films, 5 mm diameter circular samples were obtained to study the compatibility and degradation *in vivo.*

*In vivo study*

An *in vivo* study was conducted by implanting 5 samples per animal inside the bladder of adult male Wistar rats (250-300g) in aseptic conditions under general anesthesia. An incision was made on the skin and the muscle along the caudal midline with scissors to expose the bladder. After a caudal cystostomy, the samples were immobilized inside the bladder with a non-biodegradable suture (8/0). For the SHAM controls, a non-biodegradable suture was placed without any sample. The bladder was closed using a biodegradable continuous suture (8/0) and the skin and the muscle were closed using a vicryl/silk suture (4/0). After 10, 20 or 30 days, a macroscopic evaluation was done to the animals, blood and urine were collected and the animals were sacrificed for histological analysis (made at 10 and at 30 days).

Macroscopic evaluation revealed no sign of pain or affliction on the animals. The urine culture in CLED, MacConkey and blood agar was negative or not significative in all the samples. The blood study summarized in table 10 showed no alteration on the parameters studied, despite an increment on the creatin kinase values, which may be attributed to the time lapse between the extraction and the analysis. Despite the refrigeration, some samples were partially hemolyzed, which may have altered the parameters.

*Table 10: Blood analysis results at 10 and 30 days.*

| Time point | Parameter | Reference values | Control | Treatmant |
|---|---|---|---|---|
| **10 DAYS** | **Srm-creatinine** | 0.70-1.20mg/dL | 0.295 | 0.36 |
| | **San-Lactate** | 0.3-2.00nmol/L | 2.55 | 4 |
| | **Srm-proteins** | 6.6-8.7g/dL | 5.9 | 6.3 |
| | **Srm-CK (creatine kinase)** | 0-189U/L | 570.5 | 380 |
| | **Srm- Aspartate aminotransferase AST/GOT** | 0-37U/L | 81 | 102.5 |
| **30 DAYS** | **Srm-creatinine** | 0.70-1.20mg/dL | 0.29 | 0.33 |
| | **San-Lactate** | 0.3-2.00nmol/L | 2.2 | 4.05 |
| | **Srm-proteins** | 6.6-8.7g/dL | 5.9 | 6.19 |
| | **Srm-CK (creatine kinase)** | 0-189U/L | 468 | 514 |
| | **Srm- Aspartate aminotransferase AST/GOT** | 0-37U/L | 25 | 72 |

The histopathological study of the implantation site was done by counting the amount of polymorphonuclear cells (acute response), lymphocytes, plasmatic cells and macrophages (chronic response), giant cells, necrosis and fibrosis of the tissue. The histological study revealed no necrosis nor fibrosis of the tissues analyzed. Very few inflammatory cells were detected on the tissues after the surgery and the consequent implantation. Besides, as it can be seen in table 11, the highest inflammation level (level 2 out of 4) was detected at day 10 and went down at day 30 (level 1 out of 4), which indicates that the inflammation was most probably caused by the surgery process. Figure 25 shows images of the bladder after the extraction and the consequent histopathological analysis of the control versus the treatment after 10 and 30 days.

Overall, the urological, blood and histological analysis demonstrated that neither the implantation nor the degradation of the co-polymer caused any inflammatory, fibrotic or necrotic process.

*Table 11: Histopathological evaluation*

| | | Acute response | Chronic Response | | | |
|---|---|---|---|---|---|---|
| GROUP | SAMPLE | Polymorphonuclear cells (PLM) | Lymphocytes, plasmatic cells, macrophages | Giant Cells | Necrosis | Fibrosis |
| CTRL 10 DAYS | A R1 CTRL | 1 | 1 | 0 | 0 | 0 |
| | A R2 CTRL | 1 | 1 | 0 | 0 | 0 |
| | A R3 CTRL | 0 | 1 | 0 | 0 | 0 |
| | A R4 CTRL | 0 | 1 | 0 | 0 | 0 |
| | A R5 CTRL | 2 | 2 | 0 | 0 | 0 |
| TREAT 10 DAYS | A R1 POLIM | 2 | 1 | 0 | 0 | 0 |
| | A R2 POLIM | 2 | 1 | 0 | 0 | 0 |
| | A R3 POLIM | 2 | 1 | 0 | 0 | 0 |
| | A R4 POLIM | 2 | 1 | 0 | 0 | 0 |
| | A R5 POLIM | 1 | 1 | 0 | 0 | 0 |
| CTRL 30 DAYS | C R10 CTRL | 1 | 1 | 0 | 0 | 0 |
| | C R12 CTRL | 1 | 1 | 0 | 0 | 0 |
| | C R13 CTRL | 1 | 1 | 0 | 0 | 0 |
| | C R14 CTRL | 1 | 1 | 0 | 0 | 0 |
| TREAT 30 DAYs | C R1 TRAT | 1 | 1 | 0 | 0 | 0 |
| | C R2 TRAT | 1 | 1 | 0 | 0 | 0 |
| | C R3 TRAT | 1 | 1 | 0 | 0 | 0 |
| | C R4 TRAT | 1 | 1 | 0 | 0 | 0 |
| | C R5 TRAT | 1 | 1 | 0 | 0 | 0 |

The evolution of the molecular weight at the three time points (10 days, 20 days, and 30 days) of the co-polymer samples was assessed using an HPLC-GPC equipment (Metrohm, Azura, Column Linear (2) Phenogel-Phenomenex). The GPC results of the *in vivo* degradation samples are shown in Table 12. Figure 26 shows a picture of the extracted specimens on which the remnants presented a pasty form and were packed together losing its original shape. The molecular weight also decreased almost four times (at the end of the study) from its initial molecular weight (note that the molecular weight was slightly lower than the synthesized polymer as a result of the pressure melting treatment carried out to prepare the samples of the *in vivo* study).

*Table 12: Molecular weight values at different extraction times.*

| Time (days) | $M_w$ (kDa) | $M_n$ (kDa) | D |
|---|---|---|---|
| 0 | 119.5 | 63.2 | 1.92 |
| 10 | 64.6 | 36.5 | 1.77 |
| 20 | 42.2 | 23.9 | 1.79 |
| 30 | 28.1 | 18.1 | 1.55 |

## Claims

1. A rigid tubular construct suitable for insertion into a body lumen, such as the urethra, said construct comprising a polymeric material prepared by co-polymerization of ε-caprolactone, ε-decalactone, δ-valerolactone, ethylene brassylate, or δ-hexalactone with lactide,

wherein the tubular construct comprises a radially outward surface and a radially inward surface opposite the radially outward surface,
wherein the tubular construct comprises a lumen extending therethrough, the lumen being defined by the radially inward surface,
wherein the tubular construct comprises a plurality of protrusions extending radially away from the radially outward surface, and
wherein the tubular construct is not expansible.

**2.** The rigid tubular construct according to claim 1, wherein the rigid tubular construct is continuous.

**3.** The rigid tubular construct according to any one of the preceding claims, wherein said lactide is selected from L-lactide, D-lactide, racemic lactide, as well as mixtures thereof.

**4.** The rigid tubular construct according to any one of the preceding claims, wherein the number average molecular weight of the polymeric material is in the range 15 kDa to 300 kDa, such as in the range 35 kDa to 125 kDa, e.g. in the range 45 kDa to 75 kDa.

**5.** The rigid tubular construct according to any one of claims 1 to 3, wherein the weight average molecular weight of the polymeric material is in the range 15 kDa to 600 kDa, such as in the range 70 kDa to 250 kDa, e.g. in the range 90 kDa to 150 kDa.

**6.** The rigid tubular construct according to any one of the preceding claims, wherein the molar fraction of ε-caprolactone, ε-decalactone, δ-valerolactone, ethylene brassylate, or δ-hexalactone is in the range 2 to 35 of the polymeric material and the molar fraction of lactide is in the range 98 to 65 of the polymeric material.

**7.** The rigid tubular construct according to claim 6, wherein the molar fraction of ε-caprolactone, δ-valerolactone, ethylene brassylate, or δ-hexalactone is in the range 8 to 20 of the polymeric material, such as in the range 8 to 18 of the polymeric material, and the molar fraction of lactide is in the range 92 to 80 of the polymeric material, such as in the range 92 to 82 of the polymeric material.

**8.** The rigid tubular construct according to anyone of the preceding claims, wherein the ε-caprolactone, ε-decalactone, δ-valerolactone, ethylene brassylate, or δ-hexalactone is ε-caprolactone.

**9.** The rigid tubular construct according to any one of the preceding claims, wherein the tubular construct does not contain any detectable amounts of stannous octoate.

**10.** The rigid tubular construct according to any one of the preceding claims, wherein the plurality of protrusions are a plurality of circumferential rings, optionally spaced longitudinally apart from longitudinal ends of the tubular construct.

**11.** The rigid tubular construct according to claim 10, wherein each of the plurality of circumferential rings is perpendicular to the lumen and/or rounded and/or spaced longitudinally apart from adjacent ones of the plurality of circumferential rings.

**12.** The rigid tubular construct according to any one of the preceding claims, wherein the plurality of protrusions creates a corrugated structure on the radially outward surface of the tubular construct.

**13.** The rigid tubular construct according to any one of the preceding claims, wherein the tubular construct is cylindrical.

**14.** The rigid tubular construct as defined in any one of claims 1 to 13 for use in the prevention of obstruction of the urethra after surgical removal of stenosis.

**15.** The rigid tubular construct for use according to claim 14, wherein the polymeric material protects the scar tissue caused by the surgical removal of stenosis and optionally prevents urine from coming into contact with the scar tissue.

**16.** The rigid tubular construct for use according to claims 14 or 15, wherein the duration of the use is about 2 to 90 days, such as about 5 to 45 days, for example about 6 to 35 days or about 7 to 30 days.

**Patentansprüche**

1. Starre, röhrenförmige Konstruktion, die zum Einsetzen in ein Körperlumen, wie etwa die Harnröhre, geeignet ist, wobei die Konstruktion ein Polymermaterial umfasst, das durch Copolymerisation von ε-Caprolacton, ε-Decalacton, δ-Valerolacton, Ethylenbrassylat oder δ-Hexalacton mit Lactid hergestellt wird,

   wobei die röhrenförmige Konstruktion eine radial äußere Oberfläche und eine radial innere Oberfläche, der radial äußeren Oberfläche entgegengesetzt, umfasst,
   wobei die röhrenförmige Konstruktion ein sich hindurch erstreckendes Lumen umfasst, wobei das Lumen durch die radial innere Oberfläche definiert ist,
   wobei die röhrenförmige Konstruktion eine Vielzahl von Vorsprüngen umfasst, die sich radial von der radial äußeren Oberfläche weg erstrecken, und
   wobei die röhrenförmige Konstruktion nicht dehnbar ist.

2. Starre, röhrenförmige Konstruktion nach Anspruch 1, wobei die starre röhrenförmige Konstruktion durchgehend ist.

3. Starre, röhrenförmige Konstruktion nach einem der vorstehenden Ansprüche, wobei das Lactid aus L-Lactid, D-Lactid, racemischem Lactid sowie Gemischen davon ausgewählt ist.

4. Starre, röhrenförmige Konstruktion nach einem der vorstehenden Ansprüche, wobei das zahlendurchschnittliche Molekulargewicht des Polymermaterials im Bereich von 15 kDa bis 300 kDa, wie im Bereich von 35 kDa bis 125 kDa, z. B. im Bereich von 45 kDa bis 75 kDa liegt.

5. Starre, röhrenförmige Konstruktion nach einem der Ansprüche 1 bis 3, wobei das gewichtsdurchschnittliche Molekulargewicht des Polymermaterials im Bereich von 15 kDa bis 600 kDa, wie im Bereich von 70 kDa bis 250 kDa, z. B. im Bereich von 90 kDa bis 150 kDa liegt.

6. Starre, röhrenförmige Konstruktion nach einem der vorstehenden Ansprüche, wobei der Molanteil von ε-Caprolacton, ε-Decalacton, δ-Valerolacton, Ethylenbrassylat oder δ-Hexalacton im Bereich von 2 bis 35 des Polymermaterials liegt und der Molanteil von Lactid im Bereich von 98 bis 65 des Polymermaterials liegt.

7. Starre, röhrenförmige Konstruktion nach Anspruch 6, wobei der Molanteil von ε-Caprolacton, δ-Valerolacton, Ethylenbrassylat oder δ-Hexalacton im Bereich von 8 bis 20 des Polymermaterials, wie im Bereich von 8 bis 18 des Polymermaterials liegt, und der Molanteil von Lactid im Bereich von 92 bis 80 des Polymermaterials, wie im Bereich von 92 bis 82 des Polymermaterials liegt.

8. Starre, röhrenförmige Konstruktion nach einem der vorstehenden Ansprüche, wobei das ε-Caprolacton, ε-Decalacton, δ-Valerolacton, Ethylenbrassylat oder δ-Hexalacton ε-Caprolacton ist.

9. Starre, röhrenförmige Konstruktion nach einem der vorstehenden Ansprüche, wobei die röhrenförmige Konstruktion keine nachweisbaren Mengen an Zinnoctoat enthält.

10. Starre, röhrenförmige Konstruktion nach einem der vorstehenden Ansprüche, wobei die Vielzahl von Vorsprüngen eine Vielzahl von umlaufenden Ringen ist, die optional in Längsrichtung von den Längsenden der röhrenförmigen Konstruktion beabstandet sind.

11. Starre, röhrenförmige Konstruktion nach Anspruch 10, wobei jeder der Vielzahl von umlaufenden Ringen senkrecht zu dem Lumen und/oder abgerundet und/oder in Längsrichtung von angrenzenden der Vielzahl von umlaufenden Ringen beabstandet verläuft.

12. Starre, röhrenförmige Konstruktion nach einem der vorstehenden Ansprüche, wobei die Vielzahl von Vorsprüngen eine gewellte Struktur an der radial äußeren Oberfläche der röhrenförmigen Konstruktion schafft.

13. Starre, röhrenförmige Konstruktion nach einem der vorstehenden Ansprüche, wobei die röhrenförmige Konstruktion zylindrisch ist.

14. Starre, röhrenförmige Konstruktion nach einem der Ansprüche 1 bis 13, zur Verwendung beim Verhindern von Obstruktion der Harnröhre nach einer chirurgischen Entfernung von Stenose.

15. Starre, röhrenförmige Konstruktion zur Verwendung nach Anspruch 14, wobei das Polymermaterial das durch die chirurgische Entfernung der Stenose verursachte Narbengewebe schützt und optional verhindert, dass Urin mit dem Narbengewebe in Kontakt gelangt.

16. Starre, röhrenförmige Konstruktion zur Verwendung nach den Ansprüchen 14 oder 15, wobei die Dauer der Verwendung etwa 2 bis 90 Tage, wie etwa 5 bis 45 Tage, beispielsweise etwa 6 bis 35 Tage oder etwa 7 bis 30 Tage beträgt.

## Revendications

1. Construction tubulaire rigide adaptée pour une insertion dans une lumière corporelle, telle que l'urètre, ladite construction comprenant un matériau polymère préparé par copolymérisation d'ε-caprolactone, d'ε-décalactone, de 6-valérolactone, de brassylate d'éthylène ou de 6-hexalactone avec du lactide,

   dans laquelle la construction tubulaire comprend une surface radialement vers l'extérieur et une surface radialement vers l'intérieur opposée à la surface radialement vers l'extérieur,
   dans laquelle la construction tubulaire comprend une lumière s'étendant à travers celle-ci, la lumière étant définie par la surface radialement vers l'intérieur,
   dans laquelle la construction tubulaire comprend une pluralité de saillies s'étendant radialement à partir de la surface radialement vers l'extérieur, et
   dans laquelle la construction tubulaire n'est pas extensible.

2. Construction tubulaire rigide selon la revendication 1, dans laquelle la construction tubulaire rigide est continue.

3. Construction tubulaire rigide selon l'une quelconque des revendications précédentes, dans laquelle ledit lactide est choisi parmi le L-lactide, le D-lactide, le lactide racémique, ainsi que des mélanges de ceux-ci.

4. Construction tubulaire rigide selon l'une quelconque des revendications précédentes, dans laquelle la masse moléculaire moyenne en nombre du matériau polymère est dans la plage de 15 kDa à 300 kDa, telle que dans la plage de 35 kDa à 125 kDa, par exemple dans la plage de 45 kDa à 75 kDa.

5. Construction tubulaire rigide selon l'une quelconque des revendications 1 à 3, dans laquelle la masse moléculaire moyenne en masse du matériau polymère est dans la plage de 15 kDa à 600 kDa, telle que dans la plage de 70 kDa à 250 kDa, par exemple dans la plage de 90 kDa à 150 kDa.

6. Construction tubulaire rigide selon l'une quelconque des revendications précédentes, dans laquelle la fraction molaire d'ε-caprolactone, d'ε-décalactone, de δ-valérolactone, de brassylate d'éthylène ou de 6-hexalactone est dans la plage de 2 à 35 du matériau polymère, et la fraction molaire de lactide est dans la plage de 98 à 65 du matériau polymère.

7. Construction tubulaire rigide selon la revendication 6, dans laquelle la fraction molaire d'ε-caprolactone, de 6-valérolactone, de brassylate d'éthylène ou de δ-hexalactone est dans la plage de 8 à 20 du matériau polymère, telle que dans la plage de 8 à 18 du matériau polymère, et la fraction molaire de lactide est dans la plage de 92 à 80 du matériau polymère, telle que dans la plage de 92 à 82 du matériau polymère.

8. Construction tubulaire rigide selon l'une quelconque des revendications précédentes, dans laquelle l'ε-caprolactone, l'ε-décalactone, la δ-valérolactone, le brassylate d'éthylène ou la 6-hexalactone est l'ε-caprolactone.

9. Construction tubulaire rigide selon l'une quelconque des revendications précédentes, dans laquelle la construction tubulaire ne contient aucune quantité détectable d'octoate stanneux.

10. Construction tubulaire rigide selon l'une quelconque des revendications précédentes, dans laquelle la pluralité de saillies est une pluralité d'anneaux circonférentiels, facultativement espacés longitudinalement des extrémités longitudinales de la construction tubulaire.

11. Construction tubulaire rigide selon la revendication 10, dans laquelle chaque anneau circonférentiel de la pluralité d'anneaux circonférentiels est perpendiculaire à la lumière et/ou arrondi et/ou espacé longitudinalement de certains

anneaux circonférentiels adjacents de la pluralité d'anneaux circonférentiels.

**12.** Construction tubulaire rigide selon l'une quelconque des revendications précédentes, dans laquelle la pluralité de saillies crée une structure ondulée sur la surface radialement vers l'extérieur de la construction tubulaire.

**13.** Construction tubulaire rigide selon l'une quelconque des revendications précédentes, dans laquelle la construction tubulaire est cylindrique.

**14.** Construction tubulaire rigide telle que définie dans l'une quelconque des revendications 1 à 13, destinée à être utilisée dans la prévention de l'obstruction de l'urètre après une ablation chirurgicale d'une sténose.

**15.** Construction tubulaire rigide destinée à être utilisée selon la revendication 14, dans laquelle le matériau polymère protège le tissu cicatriciel causé par l'ablation chirurgicale d'une sténose et, facultativement, empêche l'urine d'entrer en contact avec le tissu cicatriciel.

**16.** Construction tubulaire rigide destinée à être utilisée selon les revendications 14 ou 15, dans laquelle la durée d'utilisation est d'environ 2 à 90 jours, telle que d'environ 5 à 45 jours, par exemple d'environ 6 à 35 jours ou d'environ 7 à 30 jours.

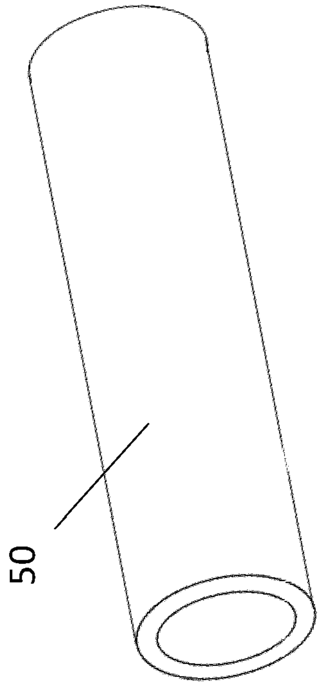


Fig. 1A

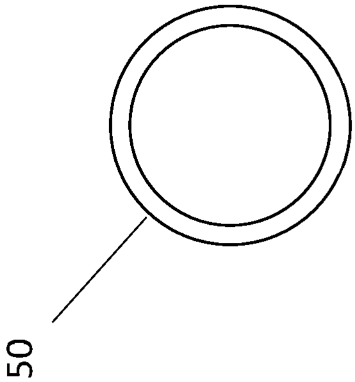


Fig. 1B

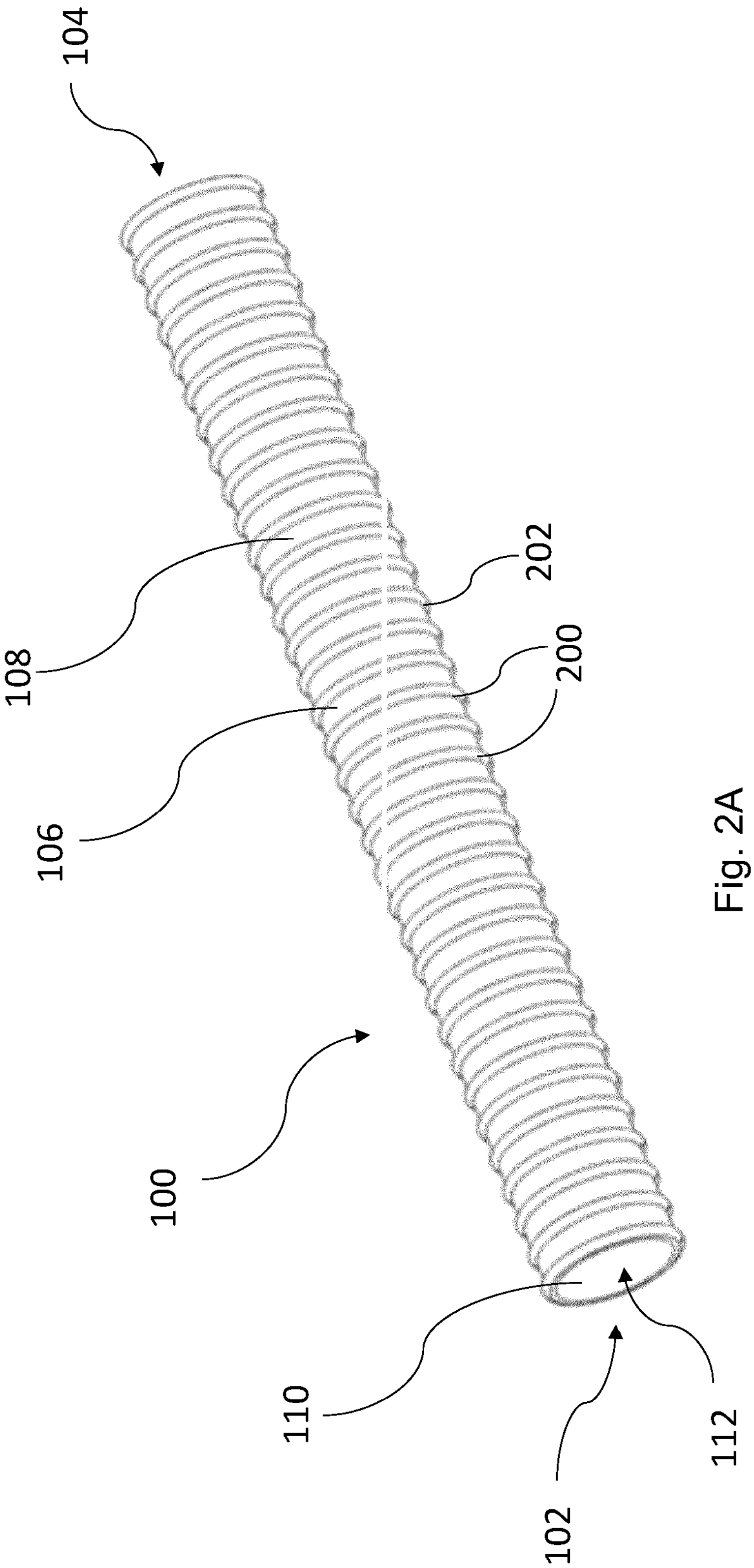


Fig. 2A

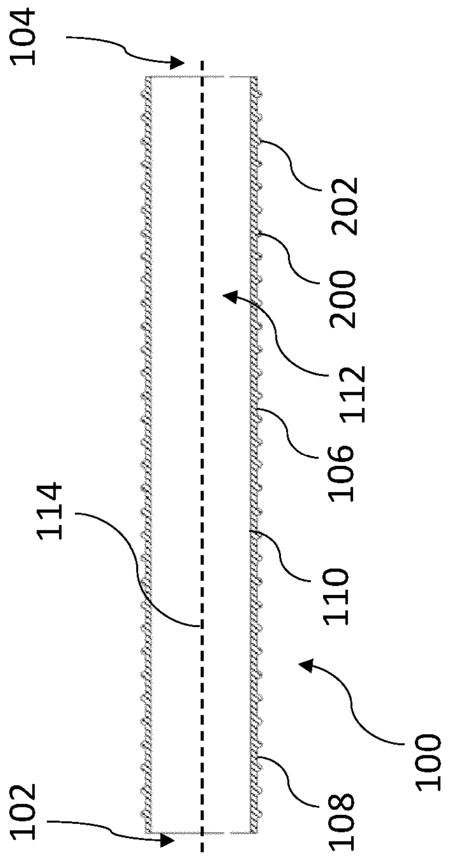
102
104
114
100
108
110
106
112
200
202

Fig. 2B

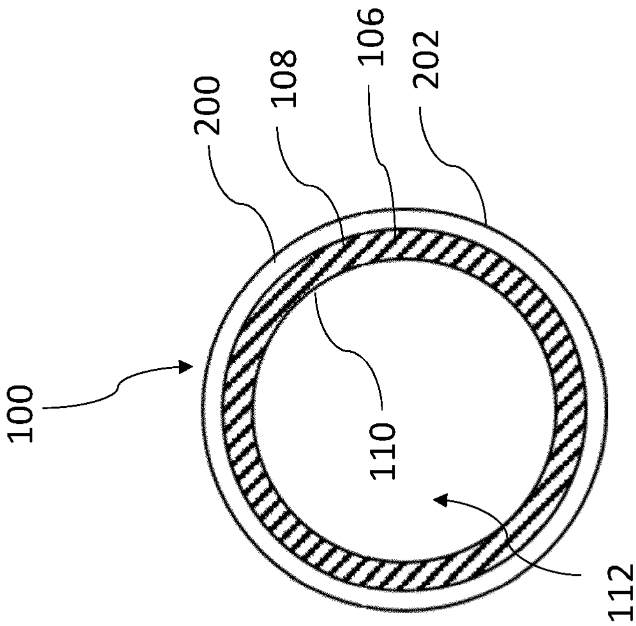
100
200
108
106
202
110
112

Fig. 2C

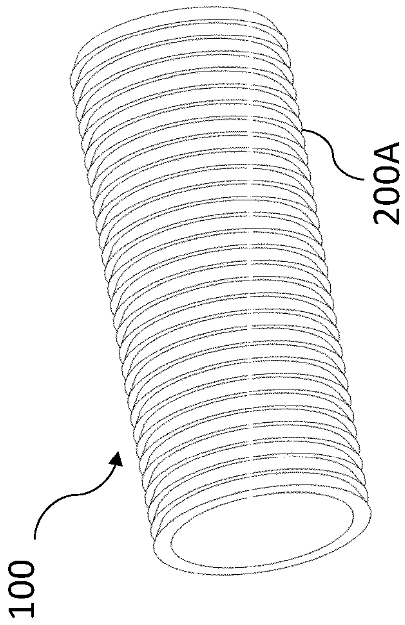


Fig. 3A

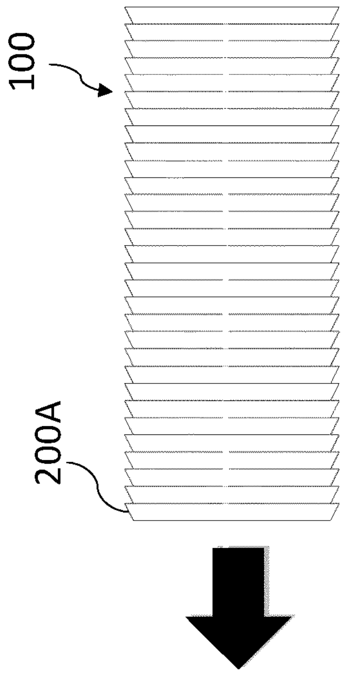


Fig. 3B

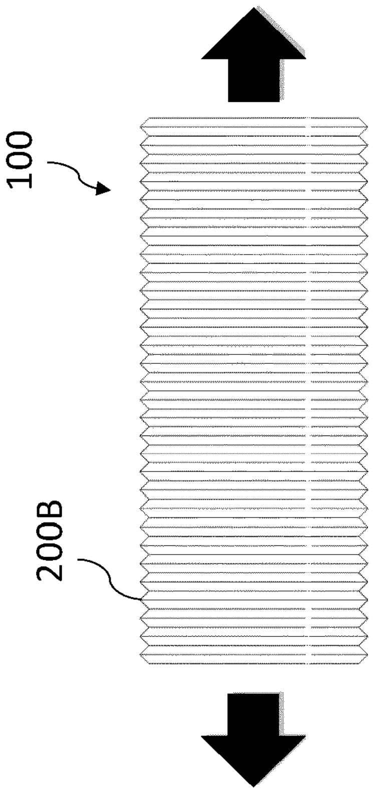


Fig. 4B

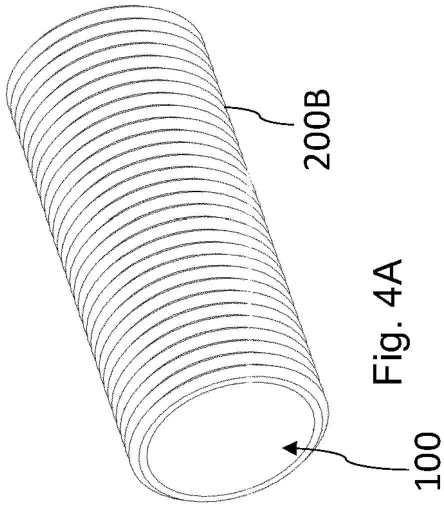


Fig. 4A

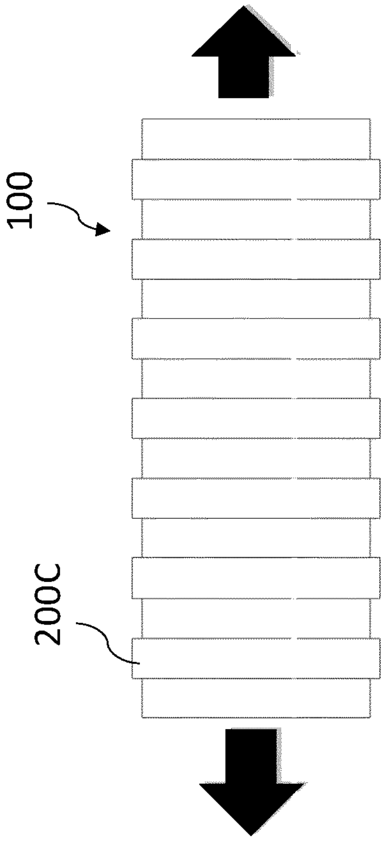


Fig. 5B

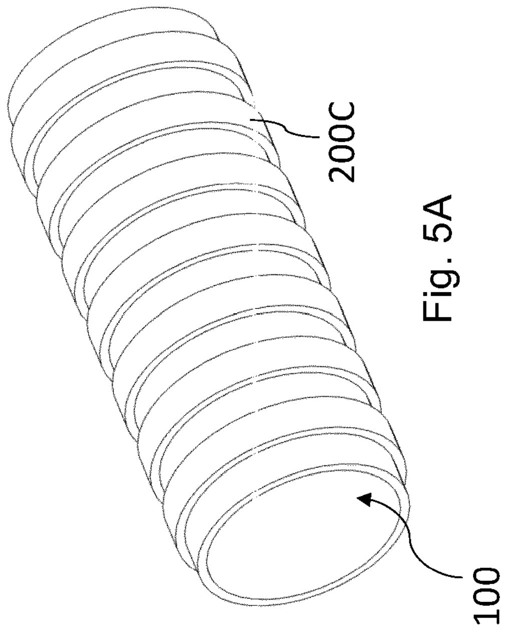


Fig. 5A

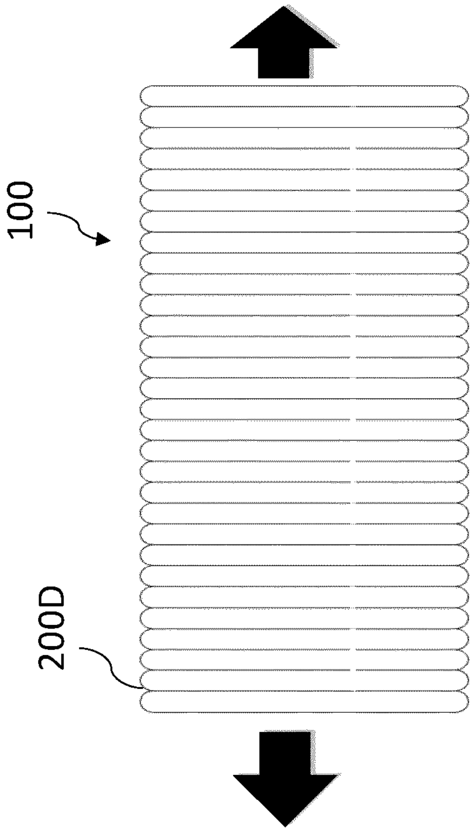


Fig. 6B

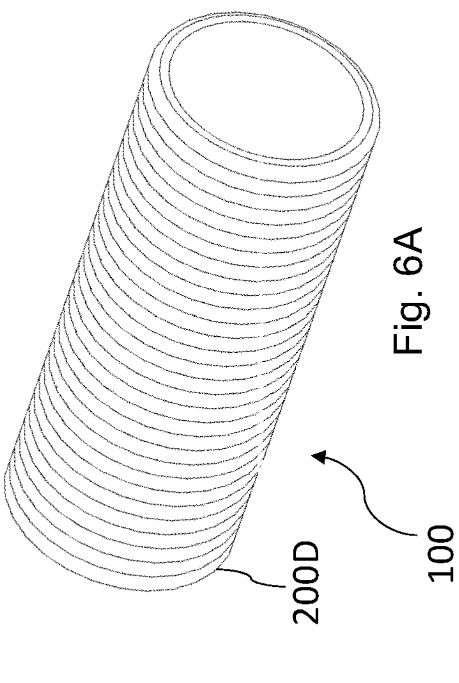


Fig. 6A

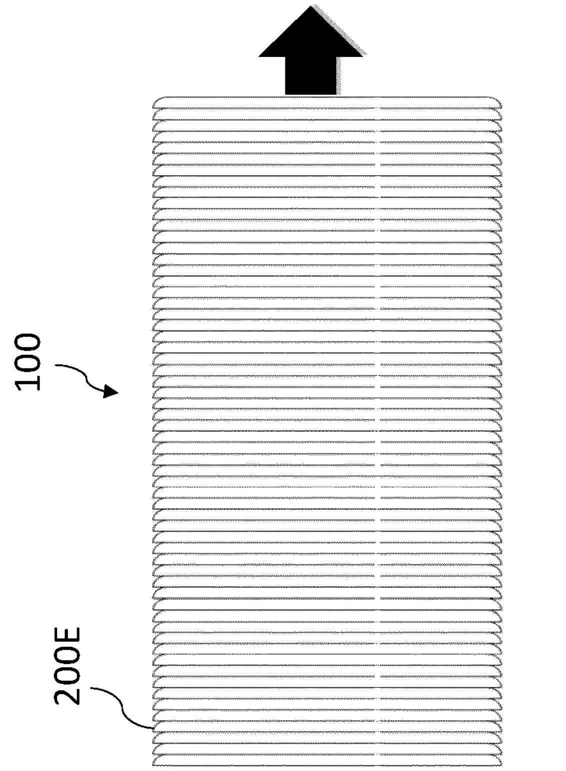


Fig.7B

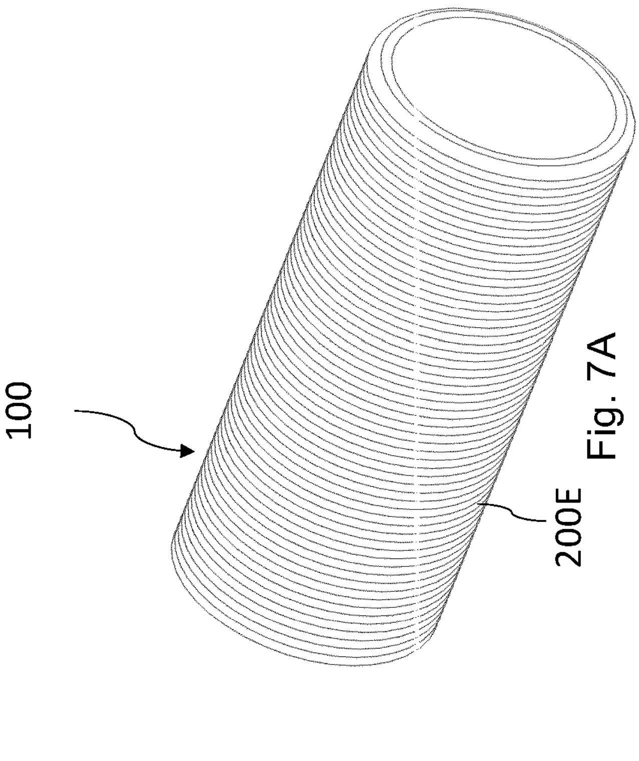


Fig. 7A

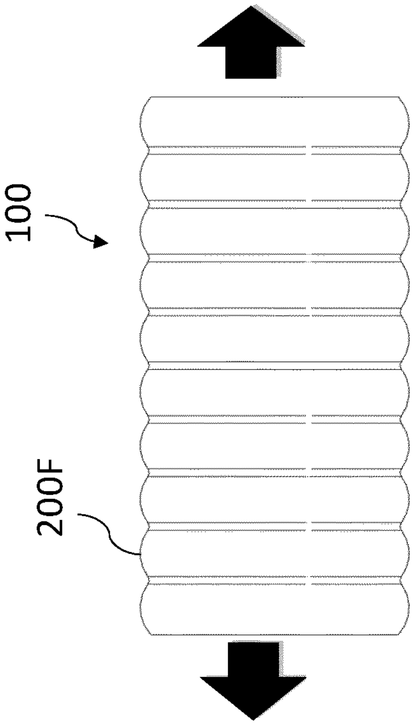


Fig. 8B

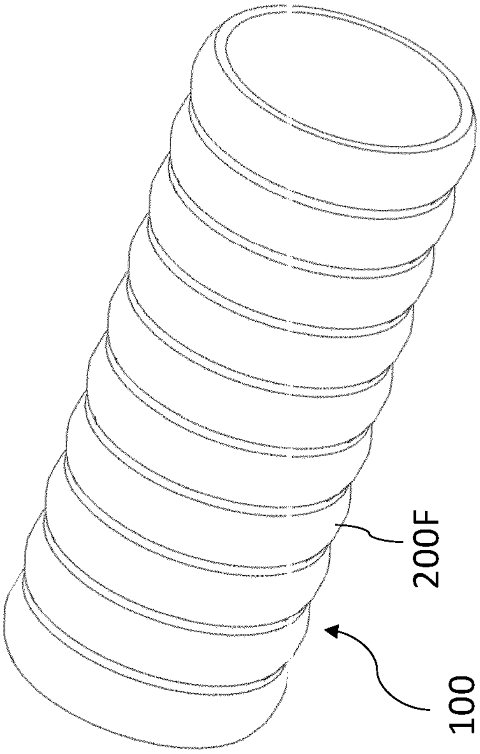


Fig. 8A

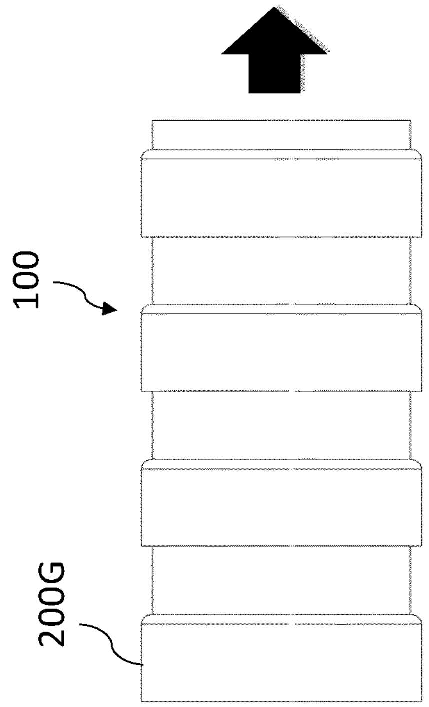


Fig. 9B

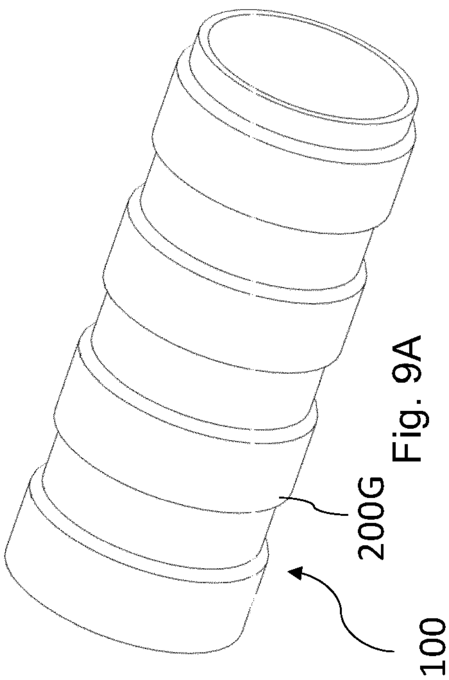


Fig. 9A

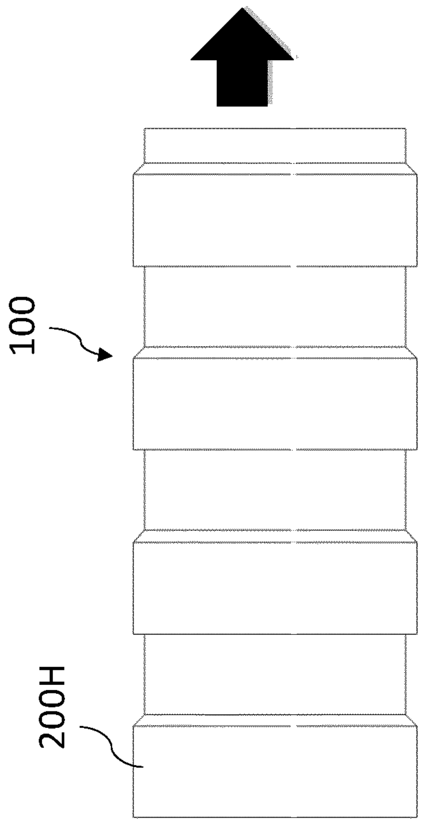


Fig. 10B

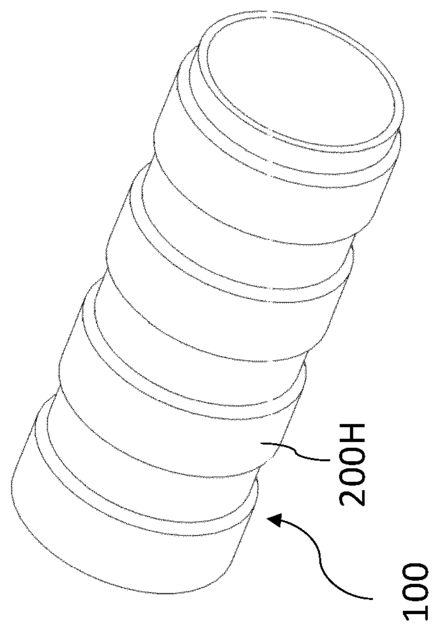


Fig. 10A

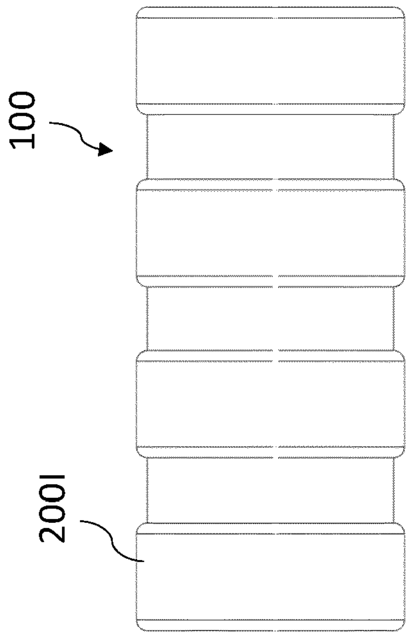


Fig. 11B

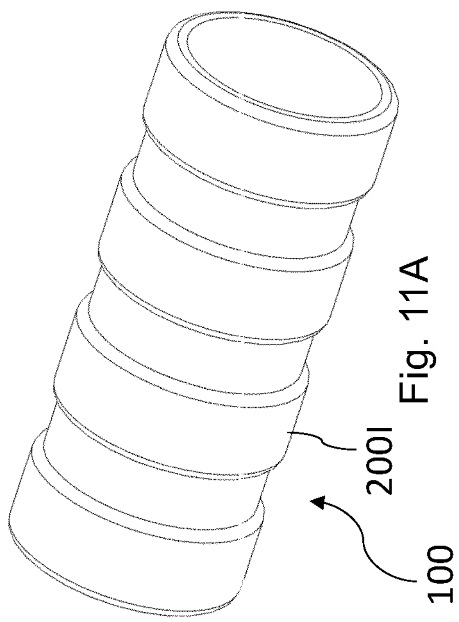


Fig. 11A

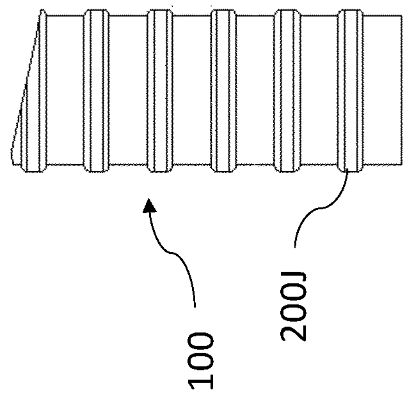


Fig. 12

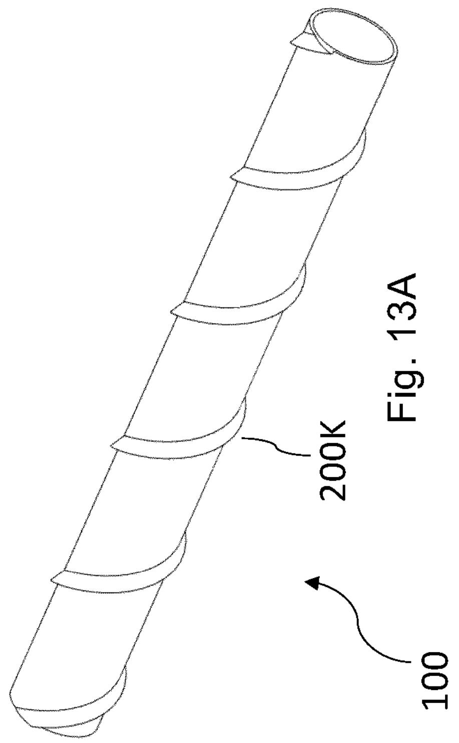


Fig. 13A

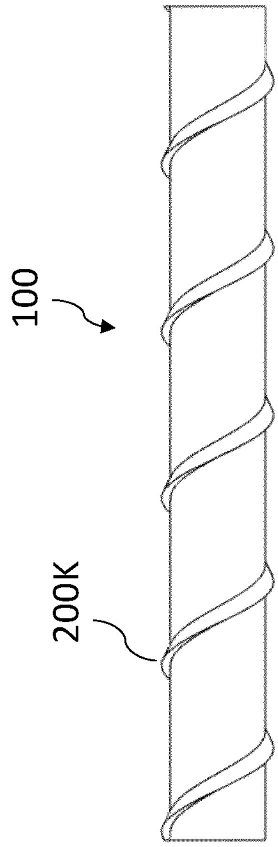


Fig. 13B

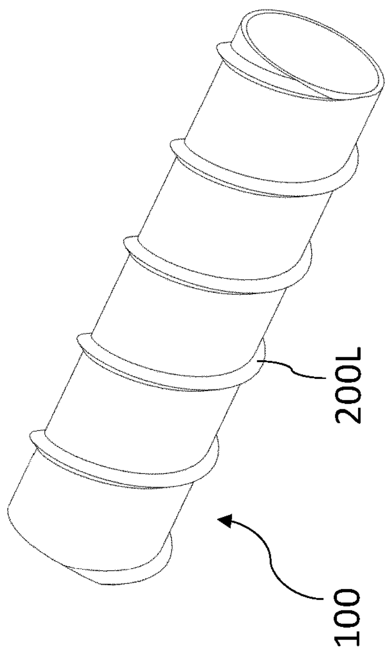


Fig. 14A

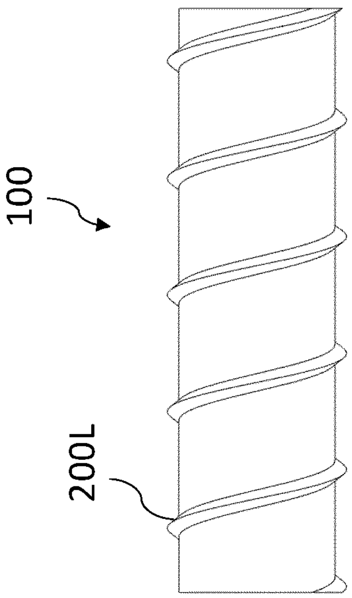


Fig. 14B

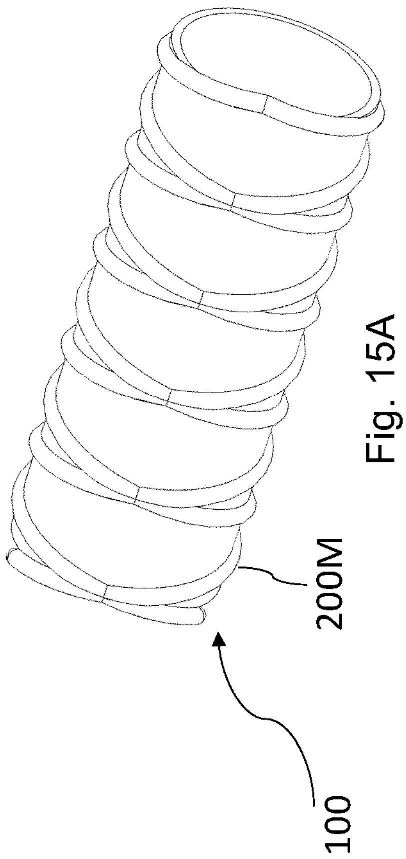


Fig. 15A

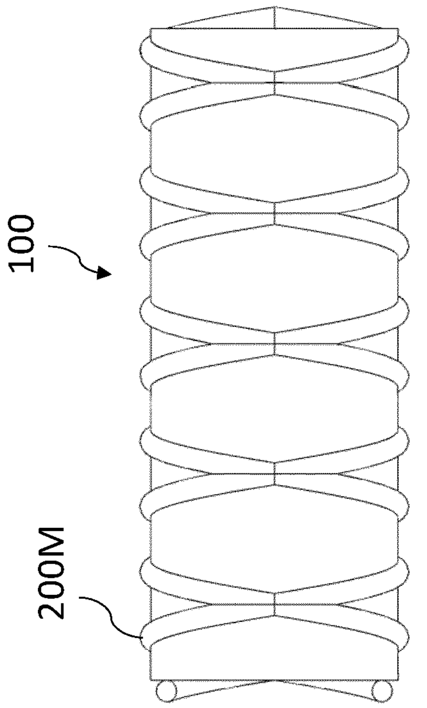


Fig. 15B

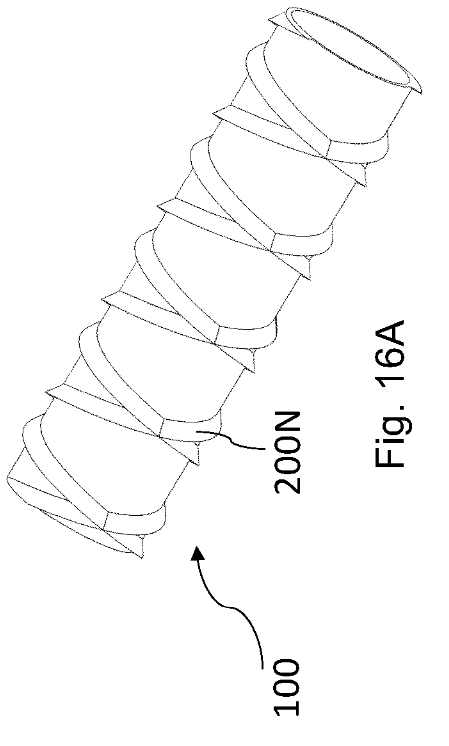


Fig. 16A

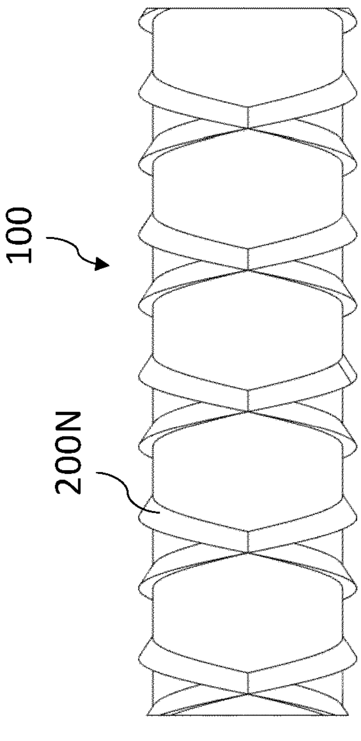


Fig. 16B

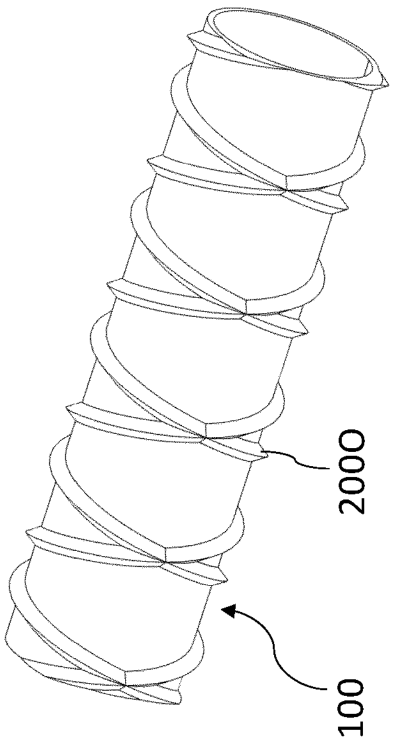


Fig. 17A

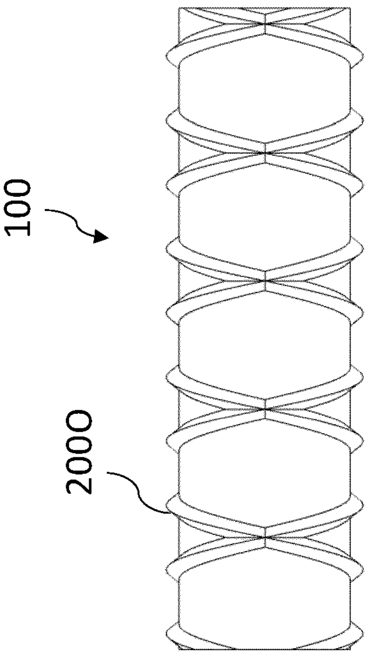


Fig. 17B

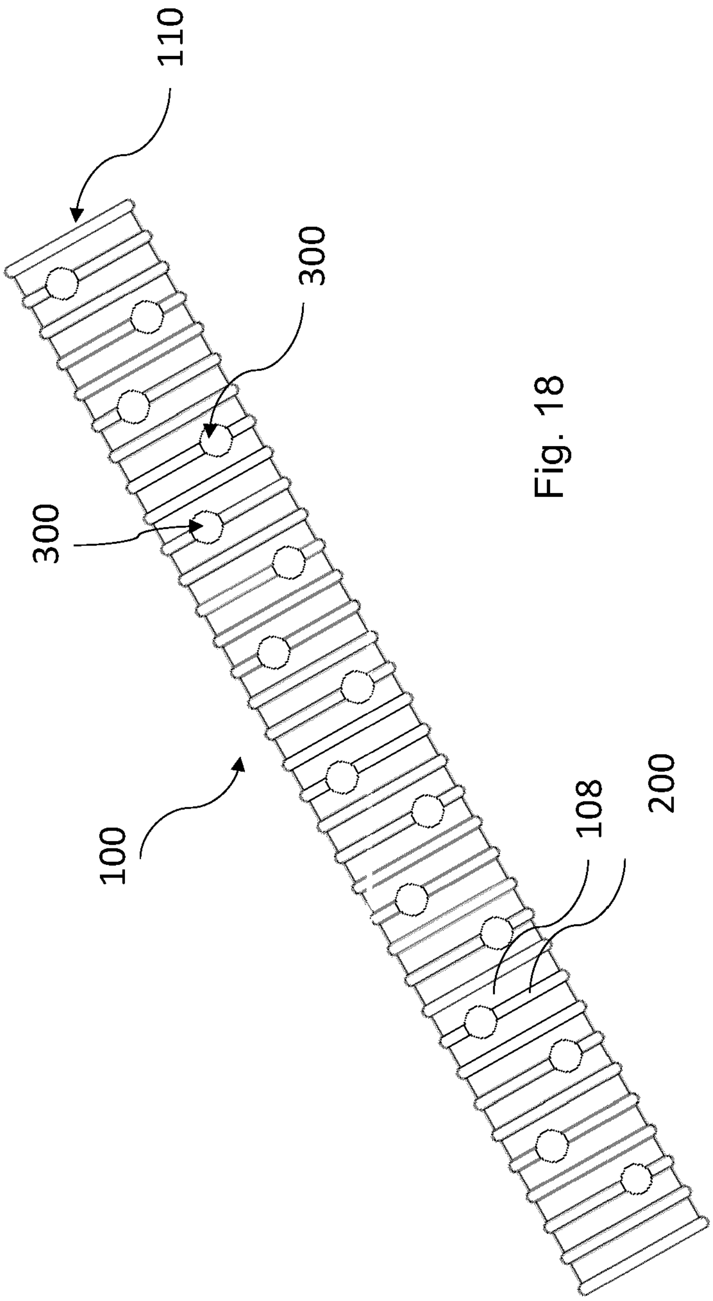


Fig. 18

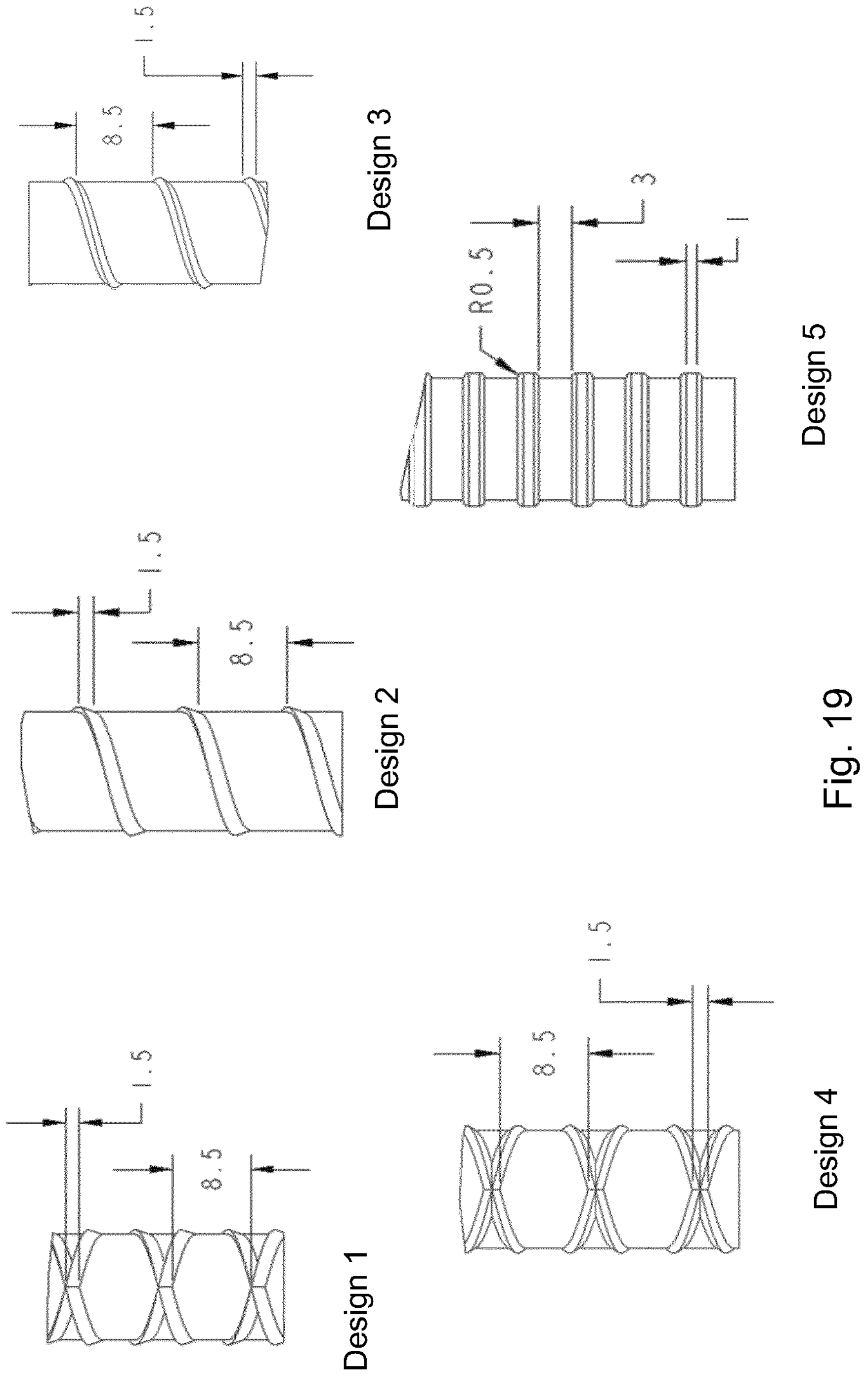
1.5
8.5
Design 3
3
R0.5
1
Design 5
1.5
8.5
Design 2
1.5
8.5
Design 1
1.5
8.5
Design 4

Fig. 19

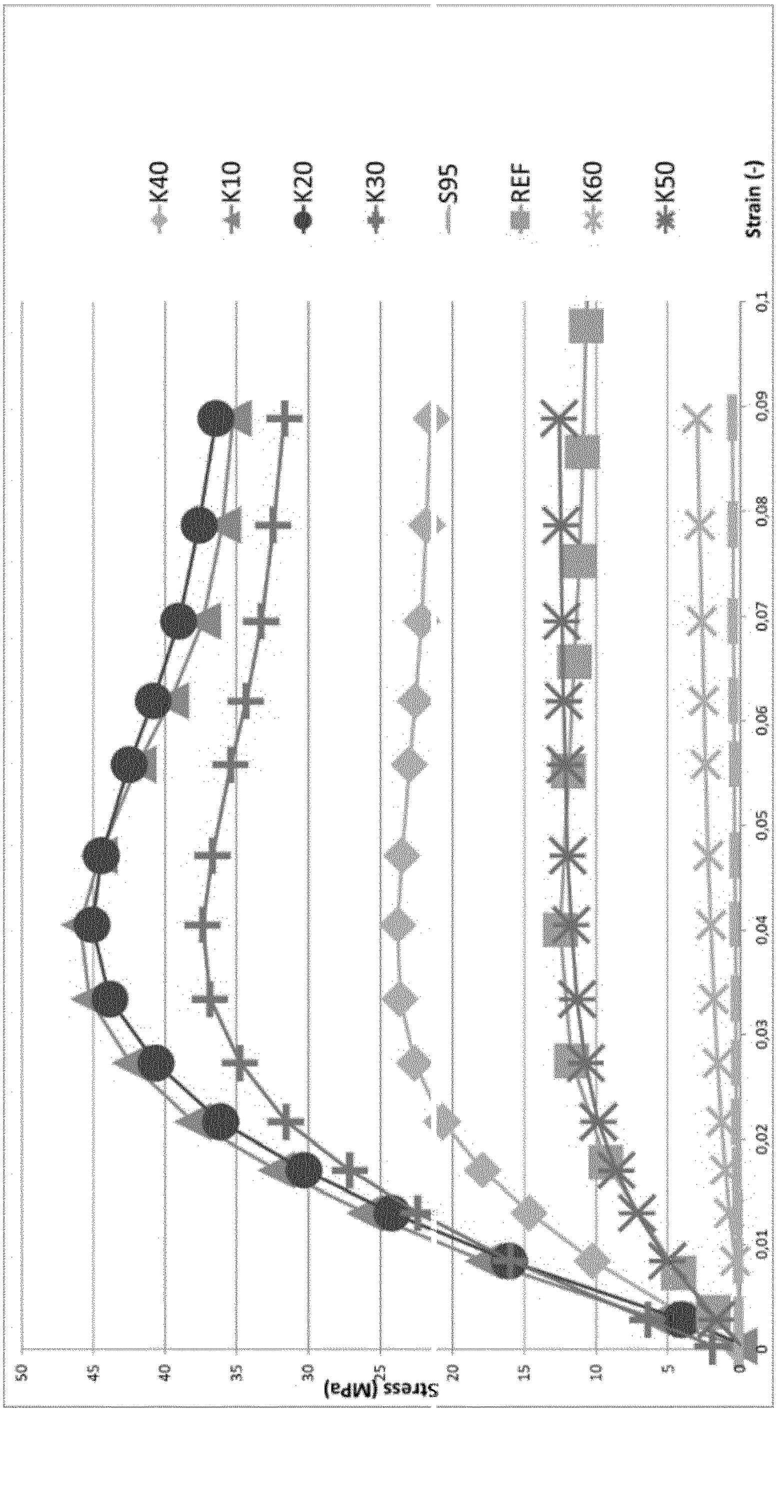
Stress (MPa)
50
45
40
35
30
25
20
15
10
5
0
0
0,01
0,02
0,03
0,04
0,05
0,06
0,07
0,08
0,09
0,1
Strain (-)
K40
K10
K20
K30
S95
REF
K60
K50

Fig. 20

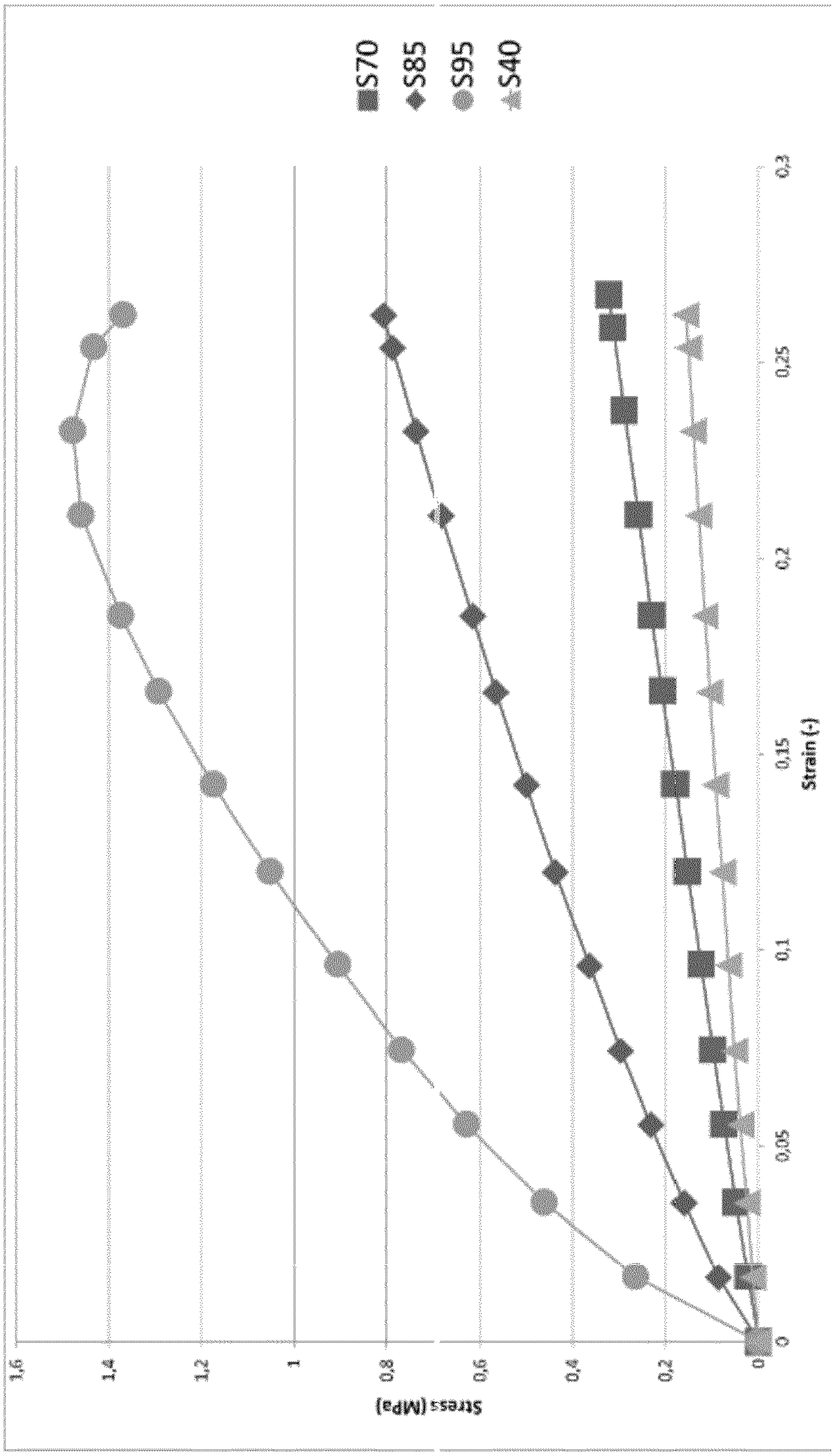

S70
S85
S95
S40
Stress (MPa)
Strain (-)
0
0,2
0,4
0,6
0,8
1
1,2
1,4
1,6
0
0,05
0,1
0,15
0,2
0,25
0,3


Fig. 21

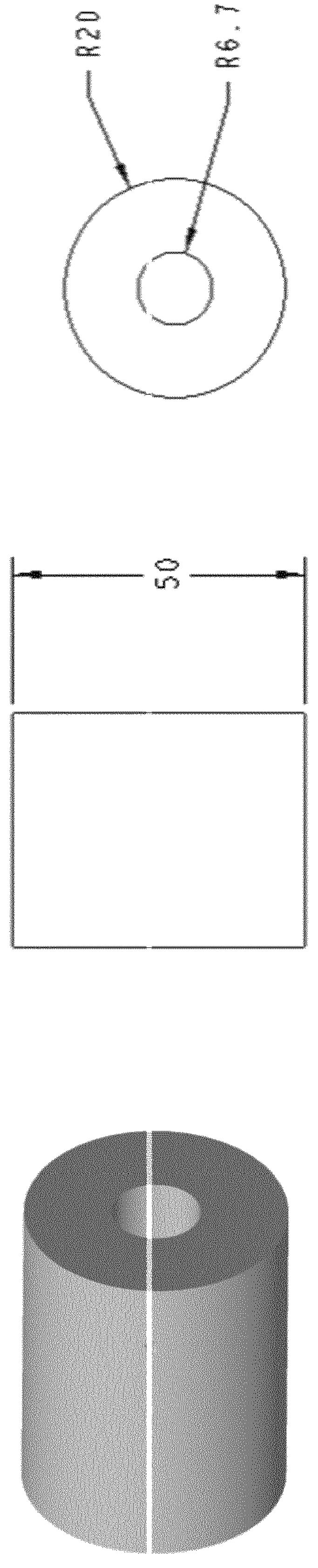


Fig. 22A

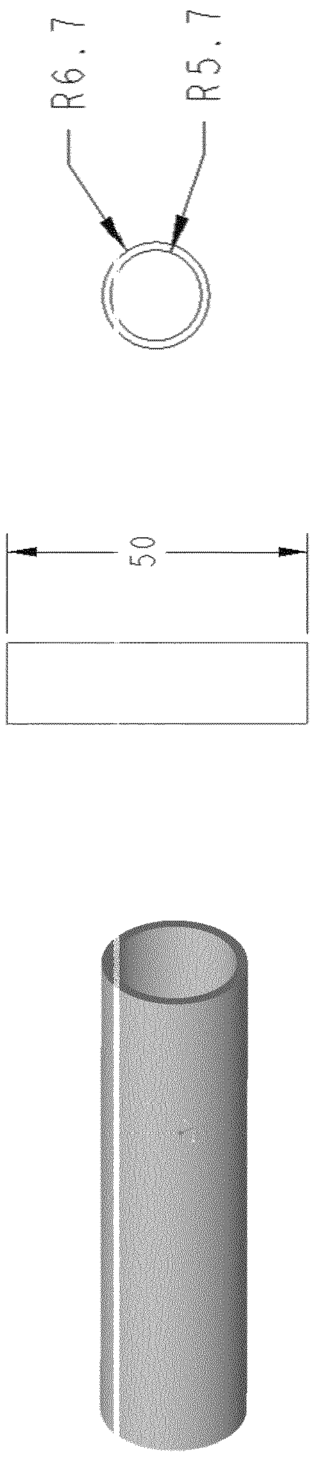


Fig. 22B

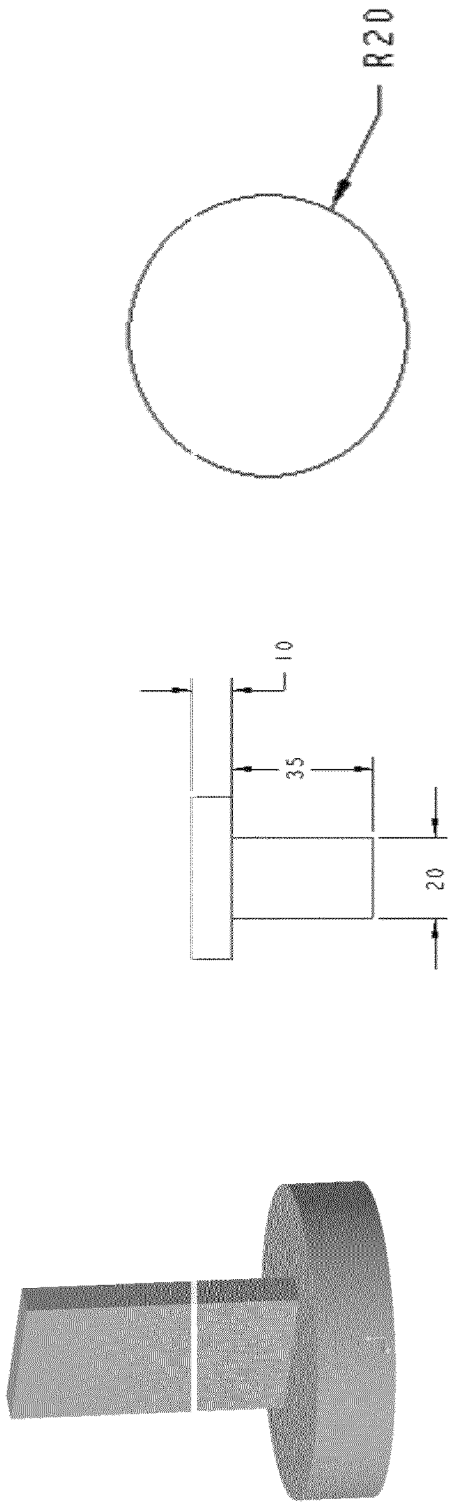


Fig. 22C

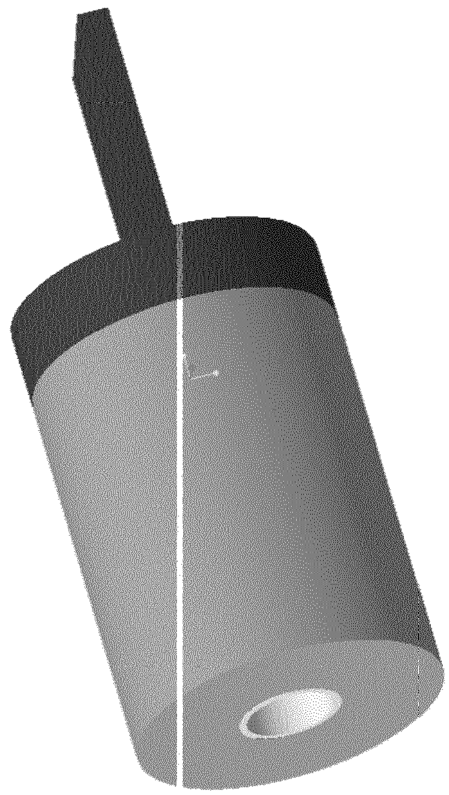

Fig. 23

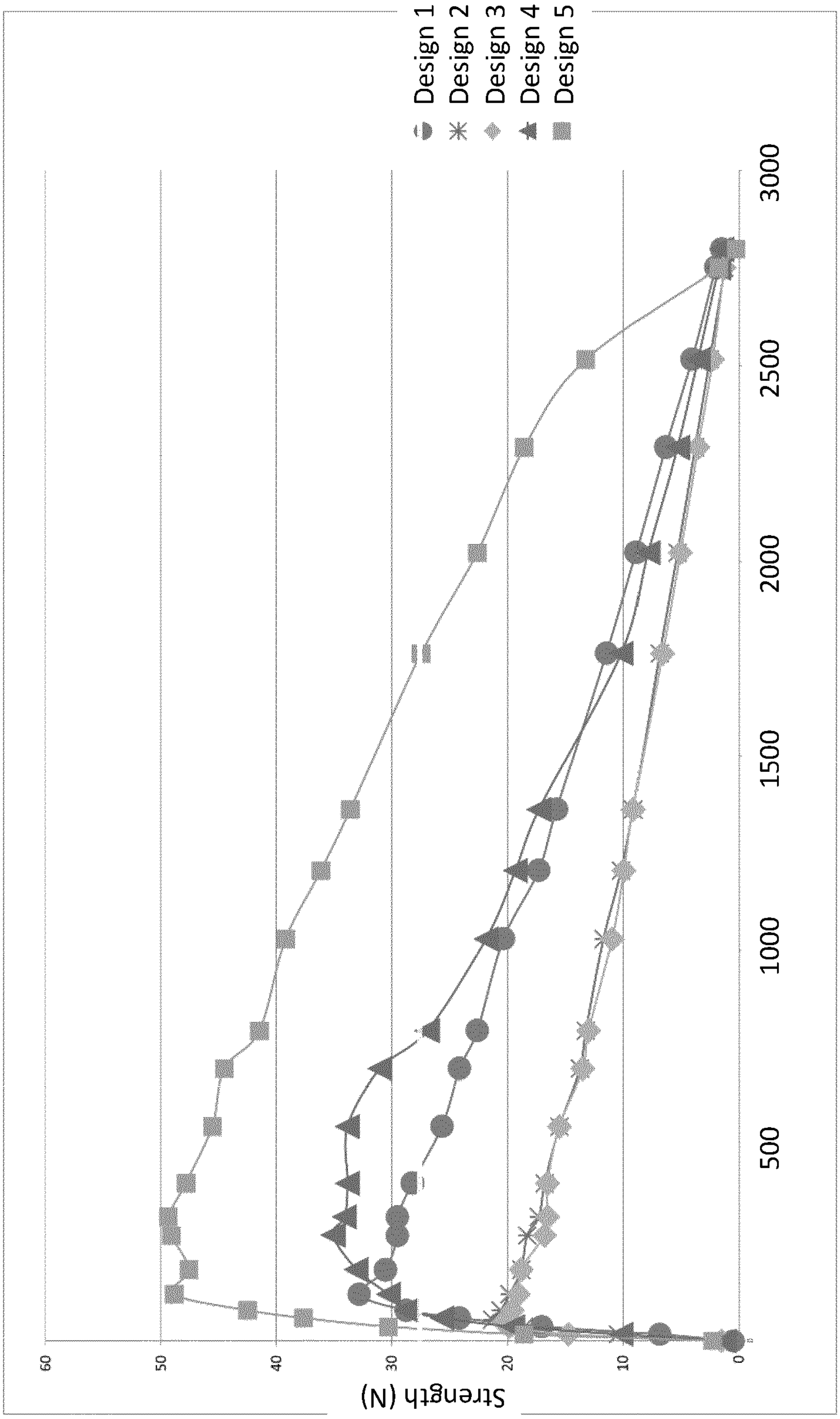
Design 1
Design 2
Design 3
Design 4
Design 5
Strength (N)
0
10
20
30
40
50
60
500
1000
1500
2000
2500
3000

Fig. 24

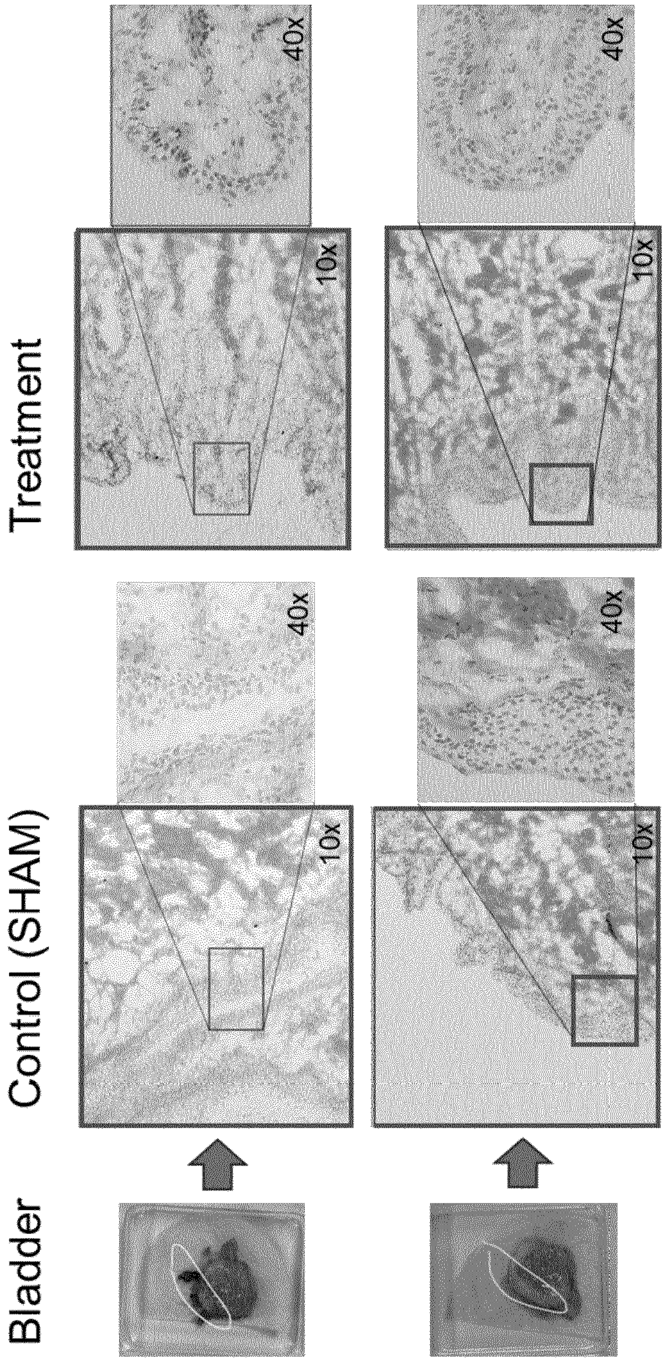
Bladder
Control (SHAM)
Treatment
10 DAYS
30 DAYS
10x
40x
10x
40x
10x
40x
10x
40x

Fig. 25

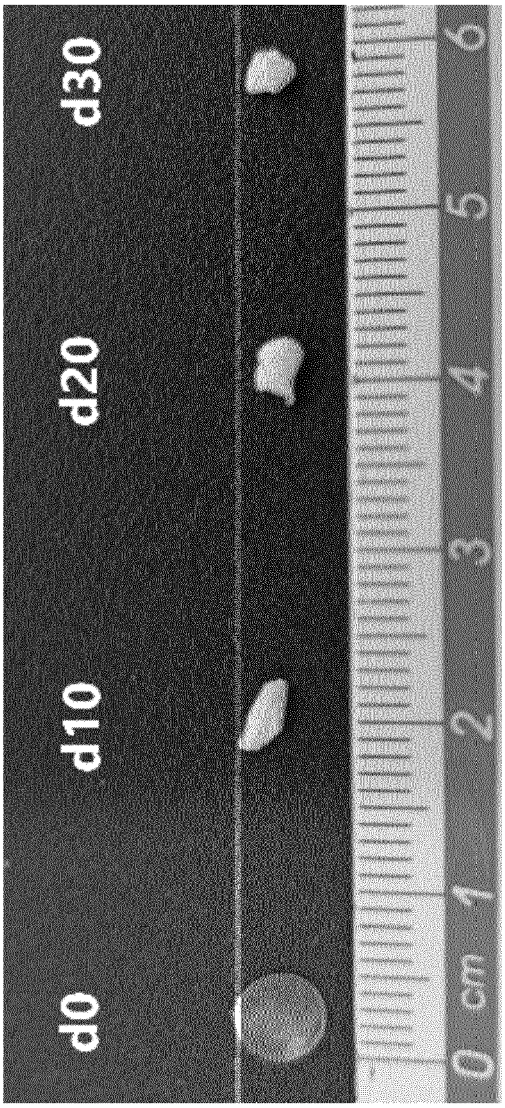


Fig. 26

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0943299 A
- US 2013331927 A1


### Non-patent literature cited in the description


- **FERNÁNDEZ, J. et al.** *Journal of the Mechanical Behavior of Biomedical materials*, 2016, vol. 64, 209-219
- **J. FERNÁNDEZ**. *Polymer Degradation and Stability*, 2017, vol. 137, 23-34
- **J. FERNÁNDEZ et al.** *Journal of the Mechanical Behavior of Biomedical Materials*, 2012, vol. 9, 100-112
- Tables of spectral data for structure determination of organic compounds. **E. PRESTCH** ; **T. CLERC** ; **J. SEIBL** ; **W. SIMON**. Chemical Laboratory Practice Book. Springer-Verlag, 2013
- Dynamic mechanical properties of oral mucosa: comparison with polymeric soft denture liners. *Journal of the Mechanical Behavior of Biomedical Materials*, vol. 4 (3), ISSN 1751-6161, 269-274